# EUROPEAN PATENT SPECIFICATION

(11) **EP 1 713 529 B1**
(45) Date of publication and mention of the grant of the patent: **24.02.2010**
(21) Application number: 05824175.3
(22) Date of filing: 28.10.2005
(51) Int. Cl.: A61M 25/02

(54) **SELF-SUTURING ANCHOR DEVICE FOR A CATHETER**
SELBSTNÄHENDE ANKERVORRICHTUNG FÜR EINEN KATHETER
DISPOSITIF D'ANCRAGE AUTO-SUTURANT PUR UNE CATHÉTER

(30) Priority: 29.10.2004 US 623502 P; 12.11.2004 US 627485 P; 16.03.2005 US 082170; 05.08.2005 US 198666; 11.08.2005 US 202484
(43) Date of publication of application: 25.10.2006
(73) Proprietor: MERIT MEDICAL SYSTEMS, INC., South Jordan, UT 84095 (US)
(72) Inventor: LAMPROPOULOS, Fred, P., Sandy, Utah 84092 (US); NELSON, Arlin, Dale, Sandy, Utah 84093 (US); BACHMAN, Darryl, Kent, Salt Lake City, Utah 84124 (US); McARTHUR, Gregory, R., Sandy, Utah 84093 (US); STOUT, Thomas, D., Salt Lake City, Utah 84115 (US); STEVENS, Brian, Pleasant Grove, Utah 84062 (US); PADILLA, William, Sandy, Utah 84092 (US)
(74) Representative: Banzer, Hans-Jörg
(86) International application number: PCT/US2005/038910
(87) International publication number: WO 2006/050080

(56) References cited:
- US-A- 4 869 719
- US-A- 4 874 380
- US-A- 5 224 935

## Description

### The Field of the Invention

Exemplary embodiments of the present invention relate to the field of catheters, and, more particularly, to a self-suturing anchor device for use with a catheter.

### Background and Relevant Art

Catheters play an important role in the treatment and care of patients in modern medicine. In particular, catheters provide relatively unobtrusive access to remote portions of a patient's body, allowing desired procedures or treatments to be performed.

A wide variety of generalized and specialized catheters have been developed and refined for particular uses. For example, angioplasty catheters have been adapted to provide a safe and effective conduit for the delivery of a stent and/or balloon to a narrowing or blockage in a patient's artery or vein. Drainage catheters are configured to be inserted into a cavity surrounding a patient's kidney, liver or other organ to drain excess fluid or infection from the cavity.

In addition, a number of devices and implements have been developed for use with catheters, to facilitate their effectiveness, or to overcome inherent difficulties associated with their use. For example, catheters that are designed to remain placed in a patient for long periods of time, such as for ongoing care or treatment of the patient, present a number of difficulties. Such catheters must be secured to the patient in a manner that minimizes movement of the catheter that could harm the patient, or otherwise interrupt proper functioning of the catheter.

Accordingly, one approach in the prior art has been to suture the catheter directly to the patient's skin. However, when a patient repositions himself/ herself in bed, the catheter may pull at the suture site or bend the catheter. Another approach is to inflate a balloon associated with the distal end of the catheter inside the patient.
However, at times an incoherent patient may attempt to withdraw or otherwise remove the catheter. This can cause injury to the catheter insertion site, or can interfere with proper operation of the catheter.

In view of these and other problems in the art, a number of devices have been developed to secure a catheter in a manner that minimizes movement of the catheter, or minimizes interference with its proper operation. Typically, such devices include an adhesive layer to be secured to the patient with a small bore for accommodating the catheter and an adhesive strip to secure the catheter relative to the adhesive layer. Devices such as these are useful because they can be employed by a practitioner to secure the desired positioning of the catheter. Such devices, however, can be undesirable due at least in part to the fact that they typically obstruct the catheter insertion site. This can make it difficult to identify infections, drainage, or other complications that may occur at the catheter insertion site. Furthermore, the devices can also obstruct cleaning of the insertion site, such that the site can only be cleaned by removing the anchor devices. Additionally, conventional anchor devices typically utilize a clip, or other securement member which typically is rigid or has a high profile when utilized to secure the catheter. As a result, the securement device can be uncomfortable if pressed against the patient by a chair, bed, or other object.

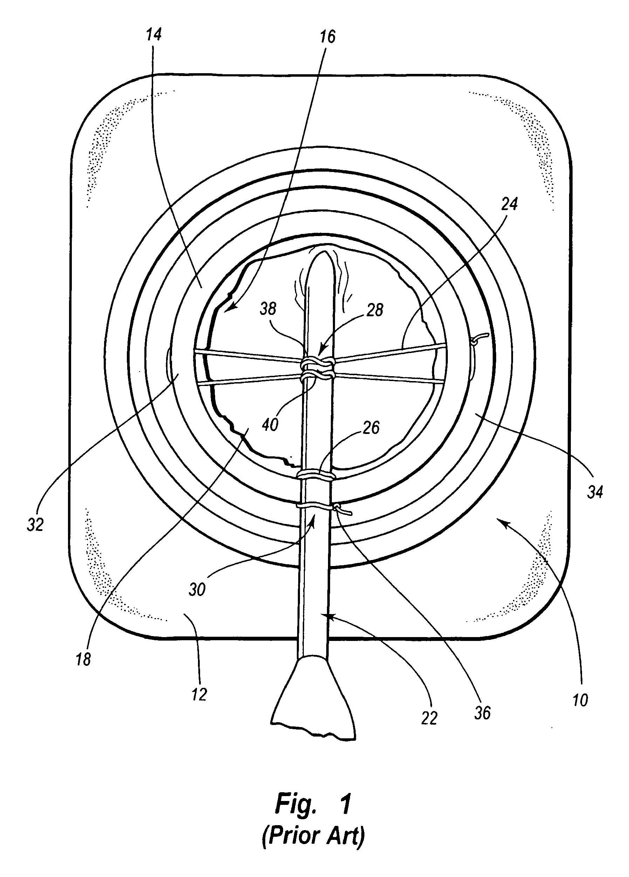

Figure 1 illustrates a modified version of an anchor device 10 that has been conventionally utilized by practitioners to secure a catheter. Anchor device 10 comprises a stoma covering that is modified by a practitioner to secure a catheter. Such stoma coverings are typically used to secure a drainage bag, such as a colostomy, illeostomy, or other similar such ostomy bag. Anchor device 10 includes an adhesive sheet 12, a semi-rigid ring 14, a sealing layer 16, and a center aperture 18.
Adhesive sheet 12 provides a mechanism for securing anchor device 10 to a patient.

Adhesive sheet 12 also secures semi-rigid ring 14 in a desired position relative to the stoma, catheter, or article or apparatus to be secured to the patient. Semi-rigid ring 14 facilitates securing of a catheter, drainage bag, or other article or apparatus.
Sealing layer 16 is positioned beneath and radially inward from semi-rigid ring 14, and covers a substantial portion of the bottom surface of anchor device 10. The inner boundary of sealing layer 16 defines center aperture 18. Sealing layer 16 provides a fluid tight seal with the patient to minimize leakage of bodily fluids outside of center aperture 18. Sealing layer 16 is somewhat resilient, or otherwise deformable, to thereby conform anchor device 10 to the patient's body around the stoma. Sealing layer 16 can include adhesive properties to maintain sealing contact with the patient.

The illustrated stoma covering also includes an adhesive backing (not shown) provided on the back side of sealing layer 16, and another adhesive backing (not shown) provided on the back side of adhesive sheet 12. The practitioner removes the adhesive backing from sealing layer 16 and adhesive sheet 12, and thereafter positions anchor device 10 on the patient's skin so that catheter 22 is within center aperture 18.

A practitioner modifies the conventional stoma covering by first removing the portion of sealing layer 16 that is positioned radially inward from semi-rigid ring 14. The portion of sealing layer 16 positioned radially inward from semi-rigid ring 14 can be removed utilizing a scalpel, scissors, or other implement. The practitioner then threads a first suture 24 from a right side 34 of semi-rigid ring 14 to a securement point 28 on catheter 22, and wraps first suture 24 around catheter 22 utilizing a first double-wrapped suture configuration 38. The practitioner then threads first suture 24 from catheter 22 to a left side 32 of semi-rigid ring 14, and then once again back to catheter 22. The practitioner then uses another double-wrapped suture configuration 40 to secure first suture 24 to catheter 22, such that the two double-wrapped suture configurations 38 and 40 define securement point 28. The second end of suture 24 is then threaded to right side 34 of semi-rigid ring 14, and tied to the first end of suture 24.

Once the practitioner has affixed suture 24 to securement point 28, the practitioner then secures catheter 22 to the bottom of semi-rigid ring 14 by first securing second suture 26 to catheter 22 adjacent bottom 36 of semi-rigid ring 14 on one side of semi-rigid ring 14. The practitioner then threads second suture 26 through the wall of semi-rigid ring 14 to an opposing side of semi-rigid ring 14. Second suture 26 is then secured to catheter 22 on the opposing side of semi-rigid ring 14. Done in this manner, catheter 22 is directly secured on both opposing surfaces of bottom 36 of semi-rigid ring 14 such that movement of the portion of catheter 22 associated with second securement point 30 is minimized.

One will appreciate, therefore, that sutures 24 and 26 can provide more stability and control of catheter 22 than would otherwise be provided by a single suture. For example, first suture 24 anchors catheter 22 to both left side 32 and to right side 34 of semi-rigid ring 14 to provide lateral stability to catheter 22 relative to a catheter insertion site. Furthermore, second suture 26 minimizes movement of the portion of catheter 22 associated with second securement point 30, such that catheter 22 does not pivot relative to securement point 28 in a manner that could result in lateral forces, pressure and/or tearing at the catheter insertion site. In addition, this arrangement allows access to the catheter insertion site and the inner boundary of semi-rigid ring 14 without obstruction. Such access allows a practitioner to identify and/or care for potential problems without needing to remove or reposition the anchor device 10.

While anchor device 10 provides advantages over other catheter securement devices, anchor device 10 presents a number of disadvantages in operation. For example, anchor device 10 is modified after the catheter is inserted into the patient. The time required to modify the anchor device and subsequently secure the catheter can add a significant amount of time to the catheter insertion procedure (e.g., 10-15 minutes or more). This can be uncomfortable for the patient in routine procedures. In more complex surgical procedures this can interfere with other aspects of the procedure to be performed, thus increasing the time the patient is under anesthesia. Additionally, the cost of the surgery is increased due to increased operating room and personnel time.

A similar catheter anchor device according to the preamble of claim 1 is known from US 5,224,935 A.

### BRIEF SUMMARY OF THE INVENTION

The present invention provides a catheter anchor device as defined in claim 1 and a method of manufacturing a catheter anchor device as defined in claim 20. The dependent claims define preferred embodiments of the invention.

The present invention relates to a self-suturing anchor device for a catheter as defined in claim 1.
The self-suturing anchor device automatically secures the catheter without requiring the practitioner to manually suture the catheter to the self-suturing anchor device. The self-suturing anchor device has a securement mechanism which is adapted to be actuated by the user to automatically secure the catheter in a quick and efficient manner.

In one embodiment, a rotatable ring is provided in connection with the self-suturing anchor device to automatically secure the catheter. A pair of suture threads extends from the bottom of the rotatable ring. When a user pulls the threads in a rearward direction, the threads automatically secure a portion of the catheter associated with the bottom of the rotatable ring. The user can then rotate the rotatable ring in one of a clock-wise or a counter clock-wise direction. Rotation of the rotatable ring automatically secures the portion of the catheter positioned centrally within the rotatable ring. In one embodiment, a locking mechanism is utilized to maintain a secured position of the rotatable ring, and thus securement of the catheter.

The configuration of the rotatable ring and the suture threads provide automatic, quick, and efficient securement of the catheter. Securement in this manner shortens the length of the procedure and simplifies securement of the catheter such that an assisting nurse can perform securement of the catheter while the physician attends to other aspects of the procedure to be performed. Additionally, in some long term care circumstances the design and operability of the anchor device is sufficiently simple that the patient, family member, or home health care provider may be authorized to change the device at home or with minimal medical supervision. In another embodiment, the securement device comprises a square shaped member having a sliding member associated therewith. In this embodiment, movement of the sliding member automatically secures the catheter in a manner as similar to rotation of the rotatable ring.

In one embodiment of the invention, opposing suture loops are embedded within a resilient pocket of the rotatable ring. The suture loops are formed with a knot or other similar member for securing one end of the suture to a middle length of the suture to form the loop. The knot or member is adapted to allow sliding of the suture thread relative to the knot (slip knot) or other loop forming member. Additionally, a portion of the loop is threaded through the knot, or member of the knot, of the other loop. Shortening of both loops by rotating the rotatable ring draws the opposing knots/ member toward opposing sides of the catheter.

The end of each suture opposite the loops is secured to a binding point on the wall of the rotatable ring. The sutures are threaded through the wall of the fixed base of the ring. Thus, when the rotatable ring is rotated, the binding point pulls the suture end of each individual suture loop along the circumference of .base of the rotatable ring. Pulling of the suture ends decreases the size of the loops resulting in loops that are smaller than the circumference of the rotatable ring. As a result, the suture loops are deployed from their embedded position in the flexible resilient pocket of the rotatable ring. As the anchor device is rotated further, the loops of each suture tighten about the catheter, thereby securing the catheter in place. Additional sutures can also be utilized with the anchor device providing additional securement of the catheter relative to the self suturing anchor device.

According to one embodiment of the present invention, the rotatable ring is utilized in connection with a ratchet mechanism. The ratchet mechanism allows movement of the rotatable ring in a first direction while preventing movement of the rotatable ring in the opposite direction. As a result, the rotational position of the rotatable ring is secured against movement in a direction that would result in loosening of the sutures. When the user rotates the rotatable ring to deploy, secure, and/or tighten the sutures relative to the catheter, inadvertent movement of the rotatable ring does not result in loosening of the sutures. Additionally, where the tension on the sutures decreases due to factors such as the natural loosening of the fibers of the suture, the user can easily ratchet the rotatable ring an additional amount to return the sutures to a desired degree of tension.

The rotatable ring can be utilized in connection with a bearing member to facilitate smooth and efficient rotation of the rotatable ring. In one embodiment, the rotatable ring comprises a stationary base and a rotatable outer ring. The stationary base is secured to the adhesive layer which is adhered to the patient. The rotatable outer ring circumscribes the stationary base allowing the outer ring to be rotated by the user relative to the stationary base. The bearing member is positioned between the stationary base and the rotatable outer ring. The bearing properties of the bearing member facilitate movement of the rotatable outer ring relative to the stationary base. In one embodiment, the bearing member includes solid surface bearing properties. In another embodiment, the bearing member comprises a bearing mechanism such as a fluid type, or roller bearing surface mechanism.

The anchor device of the present invention can -include a variety of types and configurations of features to facilitate simple and effective securement of the catheter.
For example, in one embodiment an extension saddle is utilized to provide a desired amount of displacement between suture securement points to minimize pivotal movement of the catheter. In another embodiment, the rotatable ring is wider than its height to minimize kinking of the catheter tube and to relieve pressure when pressed between the patient and a support surface. In another embodiment, a plurality of scallops or other gripping members are utilized to facilitate easy gripping and rotation of the rotatable ring. In another embodiment, a removable lid is provided which can cover the catheter insertion site such as when the user takes a shower or during sleep.

According to one embodiment of the present invention, the ratchet mechanism includes rotatable ratchet members. The rotatable ratchet members pivot allowing for movement of the portion of the rotatable ratchet member having teeth. Additionally, a ratchet member engagement spring is provided which maintains contact between the teeth of the rotatable ratchet member and teeth of the ratchet ring. The ratchet member engagement spring can flex or undergo other deformation to allow for sliding of the teeth of the rotatable ratchet member over the teeth of the ratchet ring during rotation of the rotatable ring.

In one embodiment, an O-ring is provided to maintain the position of the sutures beneath the rotatable ring. The O-ring is configured to be positioned between the rotatable outer ring and base. Maintaining the position of the sutures minimizes disruption of the sutures before deployment of the sutures. In another embodiment, the base of the anchor device comprises a single molded member. The single molded member is formed utilizing first and second mold members. The base includes an undercut. As a result, the first mold member and the second mold member are formed to provide the undercut during the molding of the base.

According to one embodiment of the present invention, a method of assembling the catheter anchor device as defined in claim 20 is provided. Loading of the sutures is facilitated by mounting the base of the anchor device on a loading block. A suture loading cylinder is positioned through the center bore of the catheter anchor device and the center aperture. The suture loading cylinder is utilized to provide a quick and effective mechanism for forming the loop configurations of the first suture, the second suture, and the third suture and for loading the sutures in the base. According to another embodiment of the present invention, one or more components of the catheter anchor device are welded to facilitate assembly of the catheter anchor device. For example, a plurality of pins of the rotatable outer ring are configured to be welded to securement bores of the bearing members. A plurality of access bores are provided in connection with the base of the anchor device such that a welding tool can be inserted through the access bores of the catheter anchor device to weld the pins of the rotatable outer ring to the securement bores of the bearing member.

According to the present invention, deployment of the securement devices can be effectuated quickly and effectively with minimal training and/or effort. Furthermore, the present invention allows a practitioner or other care provider to easily view the treatment site on the patient, thereby allowing the practitioner to treat or clean the site as needed without having to release or remove the catheter from the anchor device.

Additional features and advantages of exemplary embodiments of the invention will be set forth in the description which follows, and in part will be obvious from the description, or may be learned by the practice of such exemplary embodiments. The features and advantages of such embodiments may be realized and obtained by means of the instruments and combinations particularly pointed out in the appended claims. These and other features will become more fully apparent from the following description and appended claims, or may be learned by the practice of such exemplary embodiments as set forth hereinafter.

### BRIEF DESCRIPTION OF THE DRAWINGS

In order to describe the manner in which the above-recited and other advantages and features of the invention can be obtained, a more particular description of the invention briefly described above will be rendered by reference to specific embodiments thereof which are illustrated in the appended drawings. Understanding that these drawings depict only typical embodiments of the invention and are not therefore to be considered to be limiting of its scope, the invention will be described and explained with additional specificity and detail through the use of the accompanying drawings in which:
Figure 1 illustrates a top view of a prior art anchor device modified for securing a catheter to a patient;
Figure 2 illustrates a self-suturing anchor device in accordance with the present invention in a secured configuration after deployment of a securement mechanism;
Figure 3 illustrates the self-suturing anchor device of Figure 2 prior to deployment of the securement mechanism;
Figure 3A is an exploded cross-section view of the self-suturing anchor device of Figure 2;
Figure 3B is a perspective view of the self-suturing anchor device of Figure 2, illustrating the orientation of a first suture positioned within a resilient pocket;
Figure 3C is a perspective view of the self-suturing anchor device of Figure 2, illustrating the orientation of a second and third suture positioned within the resilient pocket;
Figure 4 is a perspective view of the self-suturing anchor device of Figure 2, illustrating deployment of a first suture;
Figure 5 illustrates the self-suturing anchor device of Figure 2, as the first suture is tightened into a double loop configuration about the catheter;
Figure 6 illustrates the self-suturing anchor device of Figure 2, after the first suture has been deployed to secure the catheter adjacent a rotatable ring of the anchor device;
Figure 7 illustrates an embodiment of the present invention in which the rotatable ring is rotated in order to deploy additional sutures to secure the catheter;
Figure 8 illustrates the self-suturing anchor device as show in Figure 7, wherein the rotatable ring is further rotated to draw additional suture loops about the catheter;
Figure 9 illustrates the self-suturing anchor device as shown in Figure 7, wherein the rotatable ring has been rotated such that the additional suture loops have tightened about the catheter;
Figure 10 illustrates an embodiment of a self-suturing anchor device having an anti-microbial patch and a saddle;
Figure 11A illustrates an embodiment of a self-suturing anchor device having a cushion layer and depicting the rotatable ring in an unlocked position;
Figure 11B illustrates the self-suturing anchor device of Figure 11A wherein rotatable ring is in a locked position;
Figure 12A illustrates an embodiment of a self-suturing anchor having a square configuration;
Figure 12B illustrates the self-suturing anchor device as shown in Figure 12A,
   in which the sutures have been deployed to secure catheter relative to the anchor device;
Figure 13 is a partial cross-sectional top view of an anchor device illustrating a catheter being secured to a patient;
Figure 14 is an exploded view of a rotatable ring of the anchor device depicting a ratchet mechanism;
Figure 15 illustrates the configuration of sutures of the anchor device utilized to secure the catheter;
Figure 16 is a top view of the anchor device of Figure 13 illustrating deployment of a first suture;
Figure 17 is a top view of the anchor device of Figure 13 illustrating deployment of a second suture and a third suture subsequent to securement of the first suture;
Figure 18 is a cross-sectional side view of a portion of the rotatable ring illustrating a bearing member of the rotatable ring;
Figure 19 is a perspective view of the anchor device of Figure 13 and a removable lid for covering the center aperture and catheter insertion side;
Figure 20 is a front perspective view of a catheter anchor device illustrating securement of the catheter subsequent to deployment of the sutures;
Figure 21 is an exploded view of the catheter anchor device illustrating the components of the catheter anchor device including an O-ring and rotatable ratchet members;
Figures 22 and 23 are perspective views of the base of the catheter anchor device illustrating molding of the base utilizing first and second mold members;
Figure 24 is an exploded view of the catheter anchor device illustrating components of the catheter anchor device that facilitate assembly of the catheter anchor device utilizing welding of one or more portions of the catheter anchor device;
Figures 25 and 26 are perspective views of the base of the catheter anchor device illustrating loading of the sutures during assembly of the catheter anchor device; and
Figures 27A and 27B are component views illustrating the ratchet mechanism including operation of a rotatable ratchet member relative to the ratchet ring.

### DETAILED DESCRIPTION OF THE PREFERRED EMBODIMENTS

The present invention extends to a self-suturing anchor device for a catheter.
The self-suturing anchor device automatically secures the catheter without requiring the practitioner to manually suture the catheter to the self-suturing anchor device. The self-suturing anchor device has a securement mechanism which is adapted to be actuated by the user to automatically secure the catheter in a quick and efficient manner.

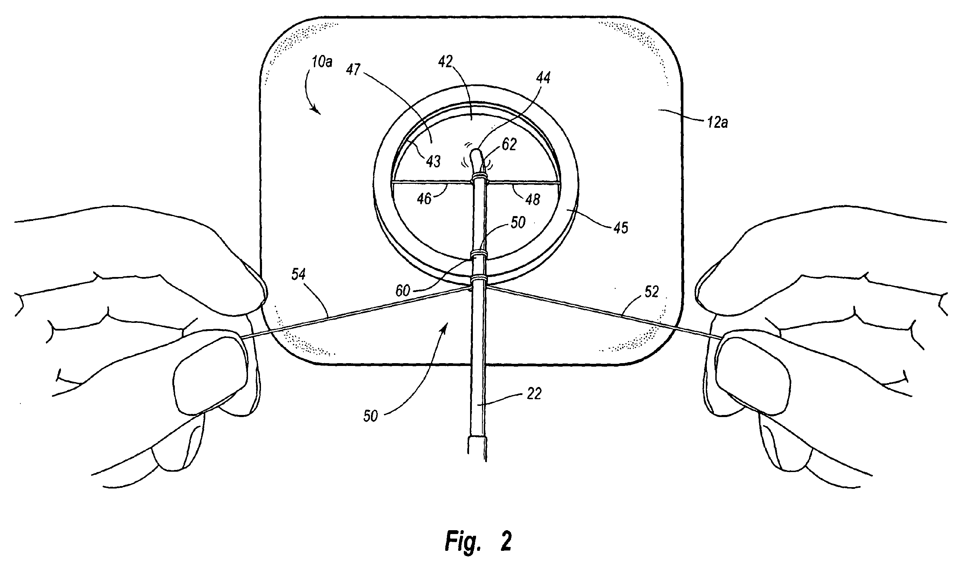

Figure 2 illustrates a self-suturing anchor device 10a having a rotatable ring 45 adapted to automatically secure a catheter 22 to self-securing anchor device 10a with a plurality of sutures 46, 48 and 50 in a quick and efficient manner. In the illustrated embodiment, catheter 22 has been inserted into and secured relative to a patient site 42. Movement of catheter 22 can cause discomfort, or even injury, to a catheter insertion site 44 of patient site 42. This can be particularly problematic when catheter insertion site 44 is at a location where catheter 22 will frequently be in contact with the patient's clothing, or in contact with a support surface, such as a chair or bed, on which the patient is resting. Self-suturing anchor device 10a of the present invention is adapted to automatically secure catheter 22 to self suturing anchor device 10a to minimize movement of catheter 22.

In the illustrated embodiment, anchor device 10a includes an adhesive sheet 12a having a central aperture 47, a rotatable ring 45, a resilient pocket 43, a first suture 50, a second suture 46, and a third suture 48. Adhesive sheet 12a is adapted to secure anchor device 10a to a patient. Rotatable ring 45 is rotatably linked to adhesive sheet 12a and associated with resilient pocket 43, as described below. Resilient pocket 43 is adapted to receive and house sutures 46, 48 and 50 therein, and allow selective deployment of sutures 46, 48 and 50 therefrom. First, second and third sutures 50, 46 and 48 are embedded within resilient pocket 43 and are adapted to secure catheter 22 to self suturing anchor device 10a. When suture 50 is pulled and rotatable ring 45 is subsequently rotated, sutures 50, 46 and 48 are deployed from resilient pocket 43 and automatically secure catheter 22 to anchor device 10a in a quick and efficient manner.

To secure catheter 22, a user first pulls a first end 52 and a second end 54 of first suture 50 to secure catheter 22 to rotatable ring 45 at a bottom securement point 60. The user then rotates rotatable ring 45 to deploy second suture 46 and third suture 48. Deployment of sutures 46 and 48 secure catheter 22 to self-suturing anchor device 10a at a center securement point 62. The present invention allows a user to quickly and efficiently secure a catheter relative to a patient. Additionally, the intuitive nature of the self suturing anchor device 10a permits the self-suturing anchor device 10a to be operated by an assisting nurse or other skilled assistant allowing the physician to attend to other aspects of the procedure.

As will be appreciated by those skilled in the art, a variety of types and configurations of self-suturing anchor devices can be utilized without departing from the scope and spirit of the present invention. For example, in one embodiment the self suturing anchor device is actuated by a mechanism other than a rotatable ring. In another embodiment, the resilient pocket does not entirely circumscribe the circumference of the rotatable ring. In another embodiment, the anchor device utilizes mechanisms other than sutures to secure the catheter. In another embodiment anchor device utilizes a plurality of resilient pockets.

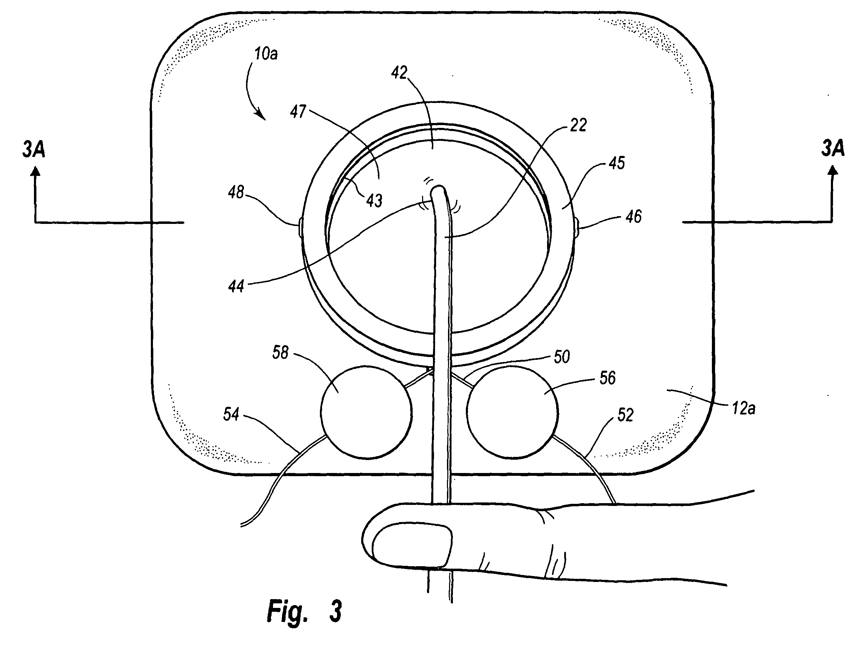

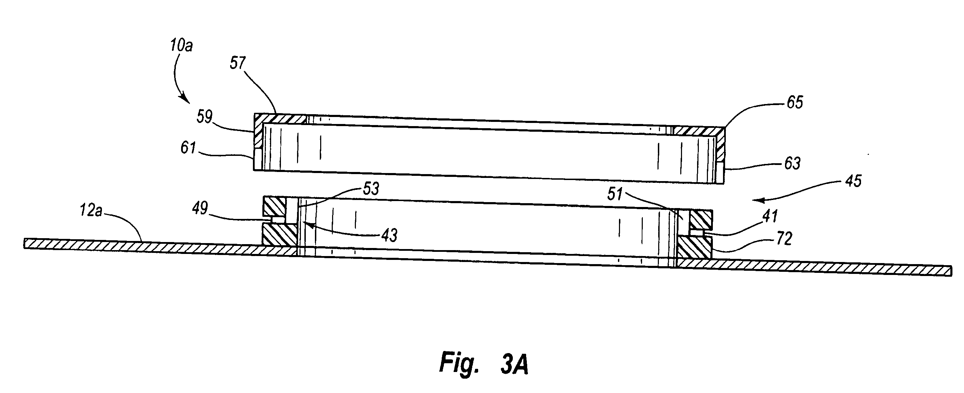

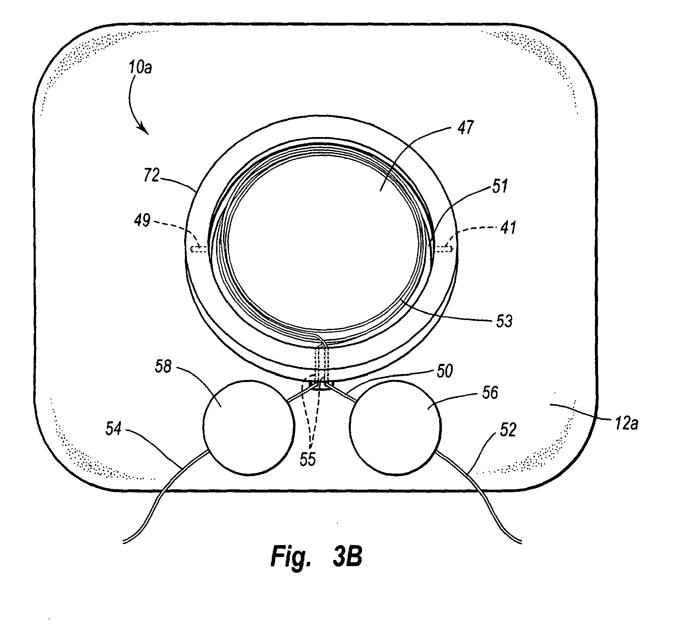

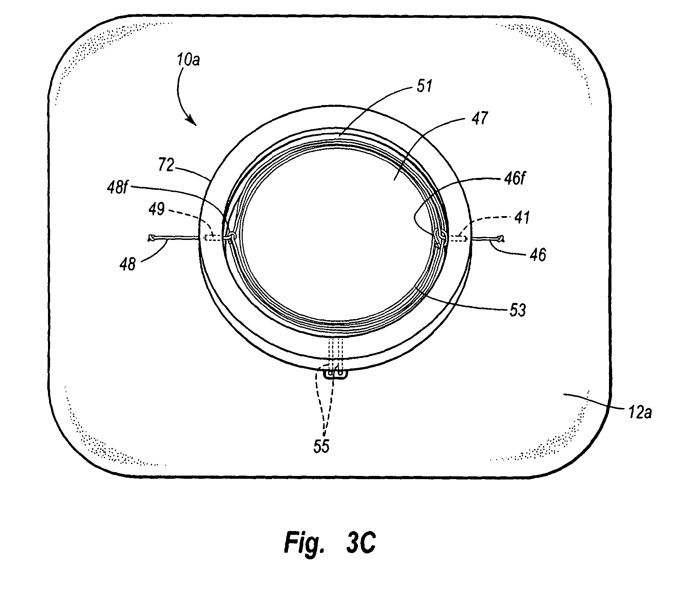

Figure 3 illustrates self-suturing anchor device 10a prior to deployment of sutures 46, 48 and 50. Central aperture 47 is adapted to allow catheter 22 to pass through anchor device 10a without obstruction. Anchor device 10a is secured to a patient with catheter insertion site 44 positioned approximately in the center of central aperture 47. Adhesive sheet 12a secures anchor device 10a to the patient to minimize movement of anchor device 10a once it is in the desired position. In the illustrated embodiment, first end 52 and second end 54 of first suture 50 are secured to adhesive sheet 12a by adhesive tabs 56 and 58. In preparation for securing catheter 22 with self-suturing anchor device 10a, a practitioner manipulates catheter 22 such that an intermediate portion of catheter 22 contacts rotatable ring 45. Such positioning of catheter 22 relative to rotatable ring 45 is advantageous for securement of catheter by sutures 46, 48 and 50 subsequent to deployment.

Figure 3A depicts an exploded cross-section view of self-suturing anchor device 10a detailing one embodiment of rotatable ring 45. In the illustrated embodiment, rotatable ring 45 comprises a rotatable outer ring 65 and a stationary inner ring, or base 72. Rotatable outer ring 65 is configured to be utilized with base 72, such that rotatable outer ring 65 can be rotated relative to base 72. Base 72 is secured to adhesive sheet 12a and includes resilient pocket 43, a first suture channel 51 (not shown), a second suture channel 41 and a third suture channel 49. Resilient pocket 43 includes a recess 51 and a resilient flange 53. Recess 51 and resilient flange 53 are configured to receive, house and retain sutures 46, 48 and 50 therein, and to allow selective deployment of sutures 46, 48 and 50 therefrom when sufficient force is exerted on sutures 46, 48 and 50. Recess 51 extends circumferentially along the periphery of base 72. Resilient flange 53 is positioned radially along the inner circumference of base 72. When rotatable outer ring 65 is positioned on base 72, resilient flange 53 is configured to retain sutures 46, 48 and 50 within recess 51 until sufficient force is exerted on sutures 46, 48 and 50. A sufficient force exerted on sutures 46, 48 and 50 causes resilient flange 53 to deflect. Deflection of resilient flange 53 allows deployment of sutures 46, 48 and 50 from recess 51.

Rotatable outer ring 65 includes a cap portion 57 and a ring portion 59. In the illustrated embodiment, ring portion 59 includes an inner surface and an outer surface concentric with the inner surface. The inner surface of rotatable outer ring 65 is configured to interface with an outer surface of base 72, such that rotatable outer ring 65 is positionable on base 72 and can rotate relative to base 72. Cap portion 57 is configured to interface with a top surface of base 72 to substantially enclose recess 51 when rotatable outer ring 65 is positioned on base 72. Rotatable ring 45 further includes a first slot 61 and an opposing second slot 63 formed in the ring portion 59 of rotatable outer ring 65. First and second slot 61 and 63 are configured to receive a suture therethrough to secure second and third suture 46 and 48 to rotatable ring 45.

Figure 3B illustrates the orientation of first suture 50 within recess 51. In the illustrated embodiment, first suture 50 is positioned within recess 51 and first suture channel 55. First suture channel 55 extends from recess 51 through base 72 to the outside surface of base 72. First suture channel 55 is configured to receive a suture therethrough. In the illustrated embodiment, first suture channel 55 includes two channels. First end 52 of first suture 50 is secured to adhesive sheet 12a by adhesive tab 56 and is received in first suture channel 55. First suture 50 is looped twice around central aperture 47 and is received back into first suture channel 55. Second end 54 of first suture 50 extends from first suture channel 55 and is secured to adhesive sheet 12a by adhesive tab 58. The orientation of first suture 50 within recess 51 facilitates deployment of first suture 50 to automatically secure a catheter.

Figure 3C illustrates the orientation of second and third suture 46 and 48 within recess 51. In the illustrated embodiment, second suture channel 41 extends from recess 51 through base 72 to the outside surface of base 72. Likewise, opposing third suture channel 49 extends from recess 51 through base 72 to the outside surface of base 72. Second and third suture channel 41 and 49 are configured to receive a suture therethrough. Second suture 46 is received in second suture channel 41, loops once around central aperture 47, and then terminates forming a slip knot 46f. A portion of second suture 46 is received within slip knot 46f to form a loop in second suture 46. Third suture 48 is received in third suture channel 49 and threaded through slip knot 46f as third suture 48 loops once around central aperture 47. Third suture 48 terminates forming a slip knot 48f. A portion of third suture 48 is threaded through slip knot 48f to form a loop in third suture 48. Additionally, a portion of second suture 46 is received within slip knot 48f. Thus, slip knots 46f and 48f each receive a portion of second suture 46 and third suture 48 therethrough. Terminal ends of second and third suture 46 and 48 are illustrated extending from suture channels 41 and 49, respectively. Terminal end of second suture 46 is configured to be received and retained within second slot 63 of rotatable ring 45 to secure second suture 46 to rotatable ring 45. Terminal end of third suture 48 is configured to be received and retained within first slot 61 of rotatable ring 45 to secure third suture 48 to rotatable ring 45. The orientation and configuration of second suture 46 and third suture 48 allow automatic securement of a catheter when rotatable ring 45 is rotated relative to base 72.

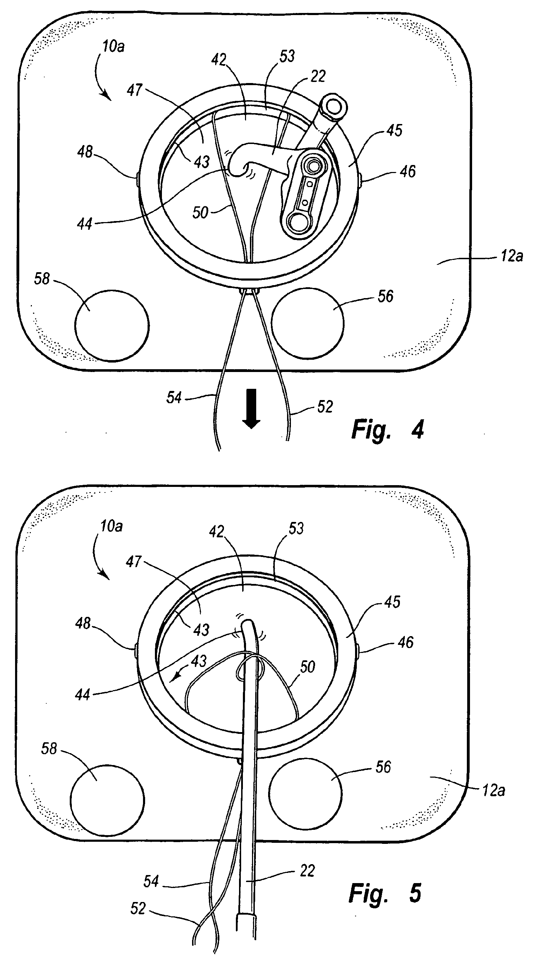

Figure 4 illustrates actuation of first suture 50 at the initialization of securing catheter 22. A practitioner first removes adhesive tabs 56 and 58. The practitioner then pulls first end 52 and second end 54 of first suture 50 in a distal direction as indicated. Pulling of the first end 52 and second end 54 in a rearward direction causes a loop portion of first suture 50 to deploy from resilient pocket 43. The loop portion of first suture 50 is configured as a double loop adapted to provide a clove-hitch type of securement arrangement around catheter 22. The illustrated position shows deployment of a single loop of the double loop of first suture 50. The first and second portions of the first loop of first suture 50 are positioned on lateral sides of catheter 22.

Resilient pocket 43 is configured to retain first suture 50 therein until sufficient force is applied to first suture 50 causing first suture 50 to deploy from resilient pocket 43. In one embodiment, resilient pocket 43 includes a retention member and an opening defined by the retention member. In this embodiment, the retention member is positioned circumferentially along the inside diameter of rotatable ring 45 adjacent to resilient pocket 43. The position of the retention member facilitates deployment of sutures 46, 48 and 50 from resilient pocket 43 through the opening defined by the retention member. The retention member is adapted to effectively prevent premature deployment of sutures 46, 48 and 50 from resilient pocket 43 while allowing deployment of sutures 46, 48 and 50 from resilient pocket 43 when sufficient force is exerted on the sutures 46, 48 and 50.

In one embodiment, the retention member includes a top and bottom resilient flange coupled to rotatable ring 45 along the inner circumference of rotatable ring 45 adjacent resilient pocket 43. The two resilient flanges are spaced apart from each other such that the distance between opposing edges of the flanges is less than the diameter of sutures 46, 48 and 50. The positioning of the two resilient flanges prevents sutures 46, 48 and 50 from inadvertently deploying from resilient pocket 43.
When sutures 46, 48 and 50 are deployed from resilient pocket 43 through the opening, a portion of at least one of the resilient flanges deflects. Deflection of resilient flanges provides an opening sufficient to allow deploying sutures 46, 48 and 50 to deploy therefrom.

Figure 5 illustrates the configuration of first suture 50 as the second loop of the double loop is being deployed. The second loop deploys as first end 52 and second end 54 are tensioned. Tensioning occurs as first end 52 and second end 54 are retracted further in the distal direction. In the illustrated embodiment, the first loop of first suture 50 has substantially tightened around catheter 22 and the first and second portions of the second loop of first suture 50 are positioned on lateral sides of catheter 22.

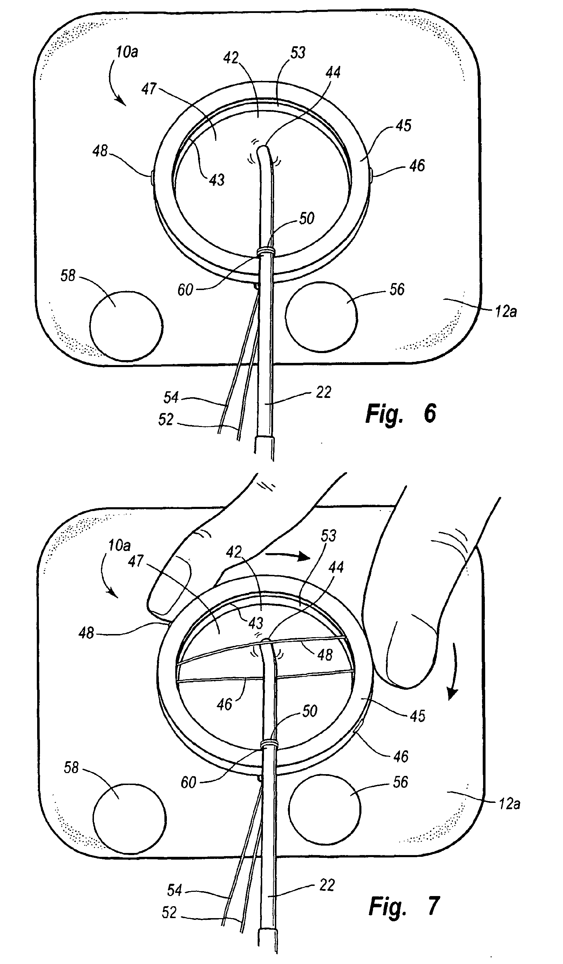

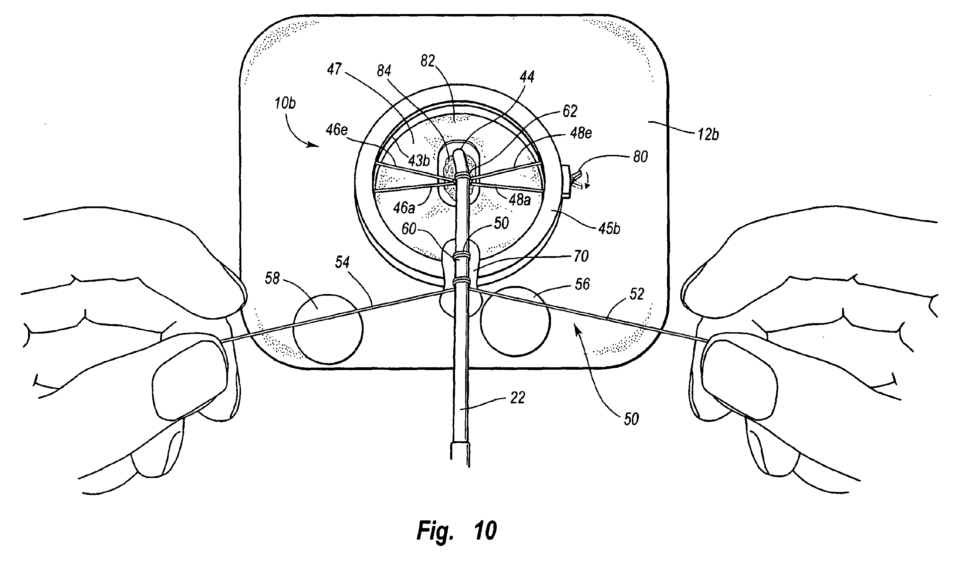

Figure 6 illustrates securement of catheter 22 by first suture 50. In the illustrated embodiment, the double loop clove-hitch configuration of first suture 50 is shown securing catheter 22 to rotatable ring 45 at a bottom securement point 60. By maintaining the tension on first end 52 and second end 54, first suture 50 retains catheter 22 against rotatable ring 45. In this embodiment, first end 52 and second end 54 are tied to catheter 22 at a subsequent step in the securement procedure, as discussed with respect to Figure 10.

As will be appreciated by those skilled in the art, the time required to secure catheter 22 utilizing first suture 50 (as depicted in Figures 3-5) can be as little as a few seconds. Not only is time saved in comparison to conventional techniques, but the steps of securing catheter 22 utilizing first suture 50 is conceptually simple, and readily conducted by a surgeon, or by an assisting nurse. As will be appreciated by those skilled in the art, a variety of types and configurations of mechanisms for securing catheter can be utilized without departing from the scope of the present invention. In one embodiment, the securement of the catheter utilizes a triple loop clove-hitch configuration. In another embodiment, the securement of catheter utilizes two double loop clove-hitch configurations from a first and second suture. In another embodiment, the suture secures the catheter utilizing an arrangement other than a clove-hitch loop. In yet another embodiment, the anchor device includes two or more resilient pockets from which multiple sutures can be deployed to secure the catheter against the rotatable ring.

Figure 7 depicts deployment of second suture 46 and third suture 48 to secure catheter 22 proximate catheter insertion site 44. To actuate sutures 46 and 48, the practitioner rotates rotatable ring 45. In the illustrated embodiment, rotatable ring 45 includes a rotatable outer portion of the ring and a stationary inner portion of the ring which provides the base of the rotatable ring 45. The practitioner rotates rotatable ring 45 in the clockwise direction approximately 1/8^{th} of a turn or 45 degrees, as shown by the displacement of the terminal end of second suture 46 relative its position in Figure 6. This rotation causes a portion of second suture 46 and third suture 48 to deploy from within resilient pocket 43 of rotatable ring 45.

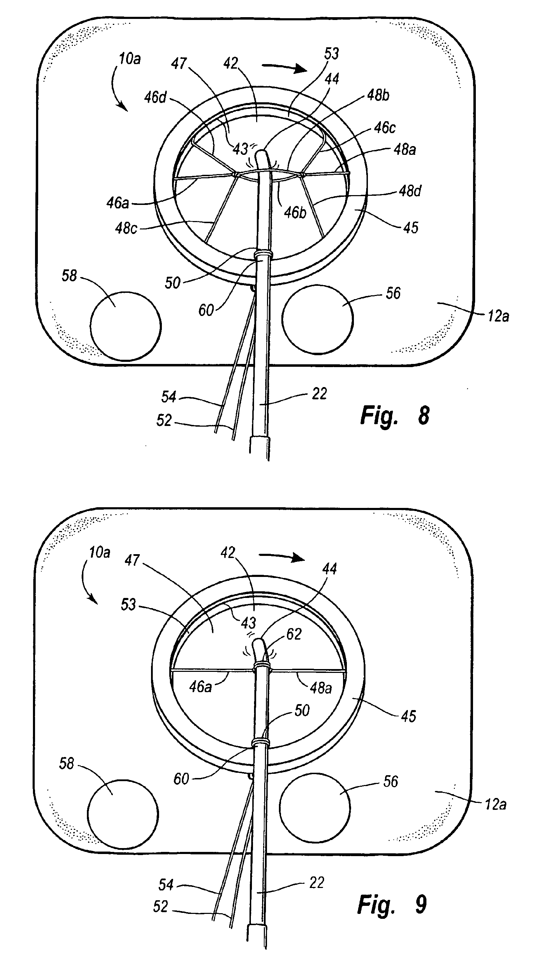

Figure 8 depicts second suture 46 and third suture 48 after additional rotation of rotatable ring 45. The loop formed by second suture 46 is represented by suture portions 46a-d of second suture 46. The loop formed by third suture 48 is represented by suture portions 48a-d of third suture 48. A portion of each of the loops of second suture 46 and third suture 48 has not been completely deployed from resilient pocket 43.

A portion of the loop of each of second suture 46 and third suture 48 are threaded through the slip knot of the opposing suture's loop. In other words, a portion of the loop of second suture 46 is threaded through the slip knot of the loop of third suture 48. Similarly, a portion of the loop of third suture 48 is threaded through the slip knot of the loop of second suture 46. The sharing of opposing slip knots in this manner allows sutures 46 and 48 to cooperatively converge around catheter 22 as they are both pulled. Additionally, the loops pull the opposing knots to converge on the catheter minimizing contact of the loops with the catheter 22 until the loops are completely tightened. This minimizes pressure that would otherwise be exerted on the catheter 22 during closing of the loops. The slip knots of second suture 46 and third suture 48 are one example of a slidable engagement mechanism utilized to form the loops of the sutures.

In the illustrated embodiment, the terminal ends of second suture 46 and third suture 48 are secured to the rotating outer ring 65 of rotatable ring 45. Rotation of rotatable ring 45 moves the terminal ends of second suture 46 and third suture 48 around the circumference of rotatable ring 45. Rotation of rotatable ring 45 pulls the ends of second suture 46 and third suture 48, thus causing the respective perimeters of the loops of both second suture 46 and third suture 48 to be reduced in length. For example, the knot at the terminal end of second suture 46 is moved from the right side of rotatable ring 45 to the left side of rotatable ring 45 during deployment of second suture 46. Similarly, the knot at the terminal end of third suture 48 is moved from the left side of rotatable ring 45 to the right side of rotatable ring 45 during deployment of third suture 48. The rotation of rotatable ring 45 pulls the ends of second suture 46 and third suture 48, further decreasing the size of the loops of second suture.46 and third suture 48. By utilizing rotatable ring 45 to deploy second suture 46 and third suture 48, the loops of second suture 46 and third suture 48 are deployed at the same time and at the same rate preventing unequal securement or unequal forces being exerted on catheter 22 during or subsequent to deployment.

As will be appreciated by those skilled in the art, a variety of types and configurations of mechanisms can be utilized in connection with deployment of the second and third sutures according to the present invention. For example, in one embodiment a slidable engagement mechanism such as a clip, ring, or preformed loop is utilized to form the loops of the first and second sutures. In another embodiment, a single suture is utilized in place of the first and second sutures. In yet another embodiment, three or more sutures are deployed during rotation of the rotatable ring. In yet another embodiment, resilient pocket 43 is formed in rotatable outer ring 65. In yet another embodiment, first and second slot 61 and 63 are positioned in base 72 of rotatable ring 45.

Figure 9 illustrates the securement of the portion of catheter 22 positioned adjacent catheter insertion site 44. The slip knot of second suture 46 is securely positioned against the left side of catheter 22. The slip knot of third suture 48 is securely positioned against the right side of catheter 22. The first and second loops formed by second suture 46 and third suture 48 encircle catheter 22 at center securement point 62. The encircling of catheter 22 at center securement point 62 by second suture 46 and third suture 48 minimizes movement of catheter 22 both laterally and in a forward and rearward direction.

As previously discussed, rotating rotatable ring 45 automatically deploys second and third suture 46 and 48 to automatically secure catheter 22 at center securement point 62. As will be appreciated by those skilled in the art, the rotation of rotatable ring 45 is a relatively straightforward task, and can be conducted in a matter of a few seconds.

Once rotatable ring 45 has been rotated sufficient for second and third suture 46 and 48 to secure catheter 22, a practitioner can then tie first arid second ends 52 and 54 of first suture 50 to finalize securement of self-suturing anchor device 10a, as illustrated in Figure 2. Self-suturing anchor device 10a of the present invention allows access to catheter insertion site 44. Access to catheter insertion site 44 allows the practitioner to ascertain the status of the catheter insertion site 44 while also permitting the practitioner, care provider, or patient to properly clean and care for the site.

Self-suturing anchor device 10a also provides a quick and effective mechanism for securing catheter 22 relative to a patient. The practitioner can affix self-suturing anchor device 10a to the patient, actuate suture 50 by pulling first end 52 and second end 54 of first suture 50, actuate sutures 46 and 48 by rotating rotatable ring 45, and then tying ends 52 and 54 of first suture 50 to secure catheter 22 relative to anchor device 10a, all in less than a minute. Additionally, the intuitive nature of actuation of anchor device 10a allows an assisting nurse or other practitioner to secure catheter 22 utilizing anchor device 10a. The present invention provides for automatic securement of catheter 22 in a quick and efficient manner by a practitioner with minimal training and/or requiring minimal effort.

As will be appreciated by those skilled in the art a variety of types and configurations of suture configurations can be utilized without departing from the scope and spirit of the present invention. In one embodiment, more than two suture connections are provided on each side of the catheter between the rotatable ring and the catheter. In another embodiment, one portion of each of the sutures are provided on each of the opposite sides of the catheter in order to provide two suture connections between the rotatable ring and the catheter. In another embodiment, a plurality of suture connections are provided between the catheter and the top and/or bottom sides of the rotatable ring. In yet another embodiment, the anchor device includes multiple resilient pockets configured to receive, house and allow selective deployment of a plurality of sutures. In this embodiment, multiple sutures are deployed from within at least one resilient pocket to secure the catheter against the rotatable ring. In another embodiment a rotatable ring having a plurality of rotatable components allows independent rotation of the plurality of components to deploy different sutures from different pockets.

Figure 10 illustrates a self-suturing anchor device 10b according to an alternative embodiment of the present invention. In the illustrated embodiment, self suturing anchor device 10b is depicted subsequent to deployment of first, second and third sutures 50, 46 and 48. Second suture 46 and third suture 48 secure catheter 22 at center securement point 62. In the illustrated embodiment, second suture 46 secures center securement point 62 relative to rotatable ring 45b utilizing second suture portions 46a and 46e. Third suture 48 secures center securement point 62 relative to rotatable ring 45b utilizing third suture portions 48a and 46e. Undesired movement of catheter is minimized by utilizing two suture portions on each side of catheter 22 to secure catheter 22 relative to rotatable ring 45b.

In the illustrated embodiment, suture portions 46a and 46e comprise two portions of the same suture thread, and are associated with a single loop portion of suture 46. Similarly, in the illustrated embodiment suture portions 48a and 48e comprise two portions of the same suture thread, and are associated with a single loop portion of suture 48. In an alternative embodiment of the present invention, suture portions 46a and 46e comprise separate suture threads, and are associated with separate loop portions, and suture portions 48a and 48e comprise separate suture threads, and are associated with separate loop portions. In the illustrated embodiment, sutures 46 and 48 are formed using 4.0 silk suture material. As will be appreciated by those skilled in the art, a variety of types and configurations of suture material can be utilized with the self-suturing anchor device.

Self-suturing anchor device 10b includes a locking lever 80, an adhesive layer 82, and an anti-microbial patch 84. Locking lever 80 is adapted to lock the rotational position of rotatable ring 45b. This allows the user to selectively lock rotatable ring 45b to maintain a desired amount of tension on sutures 46 and 48 to secure catheter 22. As will be appreciated by those skilled in the art, a variety of types and configurations of locking mechanisms can be utilized without departing from the scope and spirit of the present invention. In one embodiment, a locking button is provided that can be depressed to lock the position of rotatable ring 45b relative to other components of self-suturing anchor 10b.

Adhesive layer 82 is provided to contact the patient's skin at center aperture 47. In the illustrated embodiment, adhesive layer 82 includes a small aperture allowing a practitioner to observe catheter insertion site 44. In one embodiment, the adhesive layer 82 is transparent or semi-transparent to allow a practitioner to observe the catheter insertion site 44. Adhesive layer 82 can provide additional securement of catheter 22 relative to the patient. Additionally, anti-microbial or other properties can be imbued in adhesive layer 82 to minimize infection of insertion site 44.

Anti-microbial patch 84 is positioned adjacent catheter insertion site 44. Anti-microbial patch 84 minimizes infection at catheter insertion site 44. Anti-microbial patch 84 can also be configured to absorb blood or fluids that leak from catheter insertion site 44. Anti-microbial patch 84 can be utilized either alone or in combination with adhesive layer 82. As will be appreciated by those skilled in the art, a variety of types and configurations of adhesive covers and anti-microbial patches can be utilized without departing from the scope and spirit of the present invention.

A foam cushion 70 is shown at the point of contact between rotatable ring 45b and catheter 22. Foam cushion 70 is utilized to buffer contact between rotatable ring 45b and catheter 22. Foam cushion 70 provides a gradual flow over the upper surface of rotatable ring 45b. Furthermore, foam cushion 70 minimizes kinking, pinching, and/or other obstruction that results in the decrease of fluid flow through the drainage catheter that is caused by the contact between catheter 22 and rotatable ring 45b. Additionally, where a patient lays or rests against a surface that contacts catheter 22 at or adjacent the point of contact with rotatable ring 45b, foam cushion 70 can diffuse the pressure exerted on the patient by the combination of catheter 22 and rotatable ring 45b.

As will be appreciated by those skilled in the art, a variety of types and configurations of cushioning mechanism can be utilized with the rotatable ring without departing from the scope and spirit of the present invention. In one embodiment, a resilient rubber or gel layer is provided between the catheter and the rotatable ring. In another embodiment, the rotatable ring is flexible, resilient, or otherwise deformable to provide a layer of cushion between the patient and catheter. In another embodiment, a cushion layer is additionally provided between the rotatable ring and the patient.

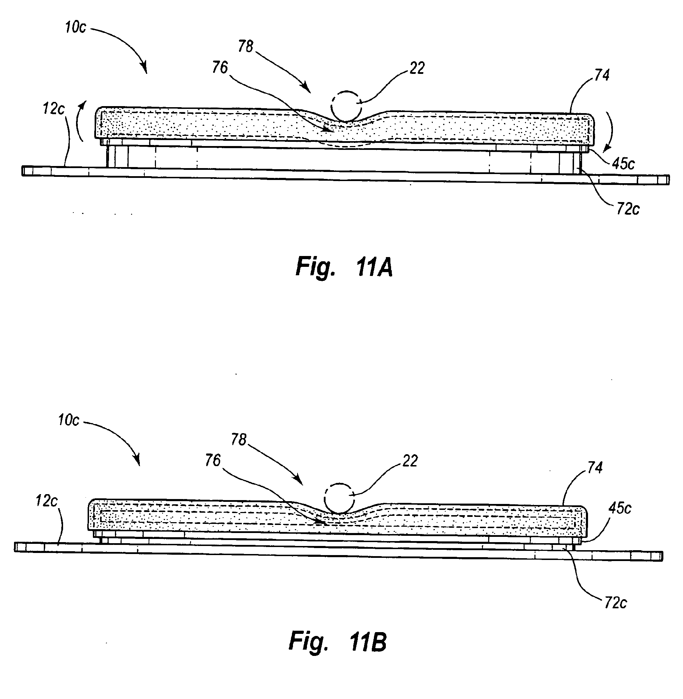

Figures 11A and 11B are side views of a self-suturing anchor device 10c according to another embodiment of the present invention. In the illustrated embodiment, rotatable ring 45c is adapted to be selectively locked to prevent rotation of rotatable ring 45c. In the illustrated embodiment, rotatable ring 45c is utilized with a stationary inner ring, or base 72c. Base 72c is secured to adhesive sheet 12c. Rotatable ring 45c can be rotated relative to base 72c. To lock rotatable ring 45c the user exerts a downward force on rotatable ring 45c such that rotatable ring 45c is pushed in a downward direction and substantially covers base 72c. When rotatable ring 45c covers base 72c as depicted in Figure 11B, rotation of rotatable ring 45c is prevented. Preventing rotation of rotatable ring 45c subsequent to securement of catheter 22 minimizes loosening of the sutures utilized to secure catheter 22. Locking rotatable ring 45c in this manner allows the user to maintain the degree and amount of securement of catheter 22 subsequent to actuation of self-suturing anchor device 10c.

As will be appreciated by those skilled in the art, a variety of types and configurations of mechanisms for preventing rotation of the rotatable ring can also be utilized without departing from the scope and spirit of the present invention. For example, in one embodiment, rotation of the rotatable ring is prevented by friction between the rotatable ring and the base. In another embodiment, rotation of the rotatable ring is prevented by interlocking engagement between the rotatable ring and the base. In yet another embodiment, the rotatable ring is configured to rotate in a ratcheting fashion, such that the rotatable ring is adapted to rotate in one direction and prevented from rotating in the other direction. In yet another embodiment, the rotatable ring is unlocked when the rotatable ring substantially covers base, and is locked when the rotatable ring is in a raised position with respect to base.

Rotatable ring 45c includes a cushion layer 74. Cushion layer 74 substantially covers and overlays rotatable ring 45c. Cushion layer 74 provides padding to minimize discomfort when a patient lays or rests on a surface in contact with self-suturing anchor device 10c. Additionally, cushion layer 74 can help to minimize kinking or pinching of catheter 22 by contact with rotatable ring 45c, or other obstruction causing a reduction of fluid flow through catheter 22. A variety of types and configurations of cushion layers can be utilized without departing from the scope of the present invention, including but not limited to resilient foam, gel, thermoplastics, rubber, latex, and the like.

In the illustrated embodiment, a saddle 76 is formed in rotatable ring 45c and a saddle 78 is formed in cushion layer 74 to accommodate catheter 22. The shape of saddles 76 and 78 minimize the exposure of catheter 22 above the upper surface of cushion layer 74 and rotatable ring 45c. Cushion layer 74 can also be deformed such that catheter 22 is minimally exposed above the upper surface of cushion layer 74. As a result, saddles 76 and 78 help minimize kinking or pinching of catheter 22, or the restriction of fluid flow through catheter 22 while decreasing discomfort that may be created by laying or contacting portions of self-suturing anchor device 10c.

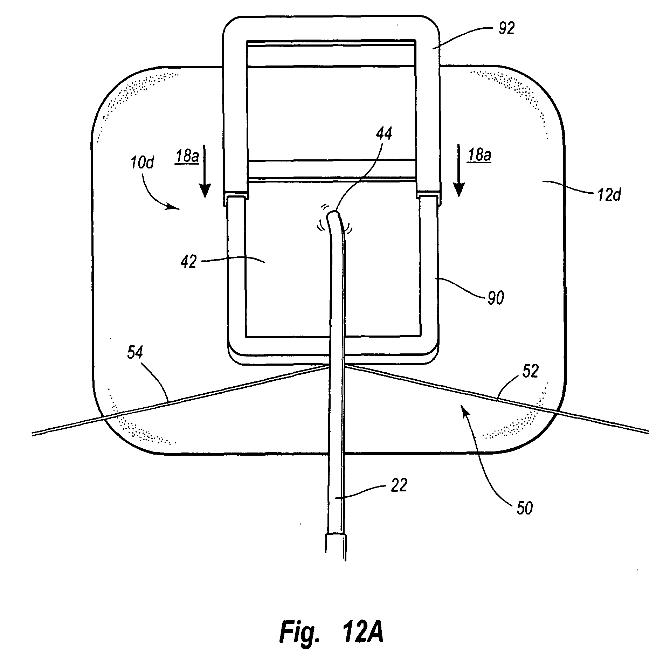

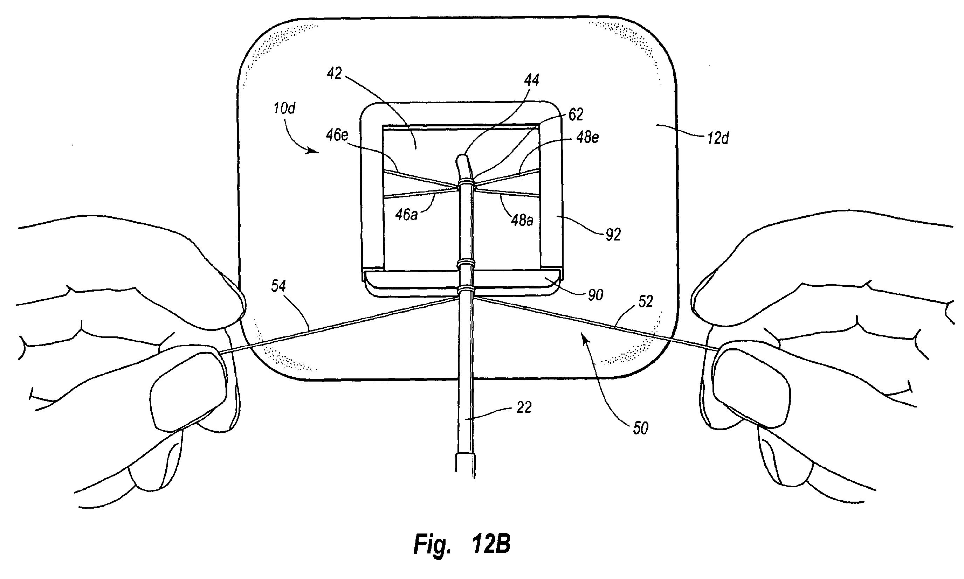

Figures 12A and 12B illustrate a self-suturing anchor device 10d according to still another embodiment of the present invention. In the illustrated embodiment, self-suturing anchor device 10d utilizes a suture deployment base 90, and a slidable portion 92. Suture deployment base 90 has a square configuration and provides a similarly-shaped aperture circumscribing catheter insertion site 44. Slidable portion 92 slidably engages the upper portion of suture deployment base 90. First suture 50 is positioned adjacent the bottom end of suture deployment base 90. First suture 50 is deployable in similar manner to that depicted and described with respect to rotatable ring 45 of Figures 4, 5 and 6.

To deploy second suture 46 and third suture 48, a user moves slidable portion 92 in the direction of directional arrows 18a. As the user moves slidable portion 92 in the direction of directional arrows 18a, sutures 46 and 48 are deployed in a manner similar to that depicted and described with respect to Figures 7, 8 and 9. The ends of suture 46 and 48 pass through the upper portion of suture deployment base 90. As a result, movement of slidable portion 92 in the direction of directional arrows 18a pulls sutures 46 and 48 along the length of the sides of suture deployment base 90. This reduces the size of the loops formed in sutures 46 and 48.

Once slidable portion 92 is moved, such that slidable portion 92 is substantially aligned with suture deployment base 90, catheter 22 is secured by sutures 46 and 48 as depicted in Figure 12B. In the illustrated embodiment a four inch by four inch (4"x4") adhesive patch (not shown) can be positioned over self-suturing anchor device 10d, to facilitate additional securement of catheter 22 relative to the patient. In one embodiment, a portion of the adhesive patch is transparent, allowing a user to view the condition of the catheter insertion site 44.

As will be appreciated by those skilled in the art, a variety of types and configurations of self-suturing anchor devices can be provided, without departing from the scope and spirit of the present invention. For example, in one embodiment, the suture deployment device is a rectangular configuration. In another embodiment, the base of the suture deployment device has a different shape than the rotatable or slidable member. In another embodiment, the adhesive sheet is circular, rectangular, or of a different size or configuration. In still another embodiment, the size of the adhesive sheet utilized to overlay the suture deployment device varies. In yet still another embodiment, the type and configuration of sutures utilized to secure the catheter are adapted to the type of catheter to be secured, and can be varied. In another embodiment, the self-suturing anchor device comprises both a slidable portion and a rotatable portion used in conjunction to comprise the suture deployment device.

In general, securement means are directed primarily to sutures, strings, cords, wires, bands, adhesive strips, rigid elements, or other securement mechanisms that are configured to tighten one or more sutures about a first securement point of a catheter by a user actuating at least a portion of the securement means. The securement means can also include specific device elements configured to tension one or more sutures to secure a second securement point of the catheter against a portion of the catheter anchor device.

For example, to accomplish the function of securement, the relevant device means can include a rotatable ring (e.g., 45) where one of the sutures (e.g., 46 or 48) is secured to a portion of the rotatable ring on one end, and also secured to itself (e.g., with a slip knot joining suture portions 46a, 46d, and 48c, or suture portions 46c, 48a, and 48d) on an opposing end inside a resilient pocket (e.g., 43). Accordingly, the securement means provide that, as the one end (e.g., 46, 48) is moved, the corresponding suture (e.g., 46a-e, 48a-e) is configured to come out of the one or more resilient pockets (e.g., 43), and slidably tighten about a catheter 22 positioned inside the central aperture.

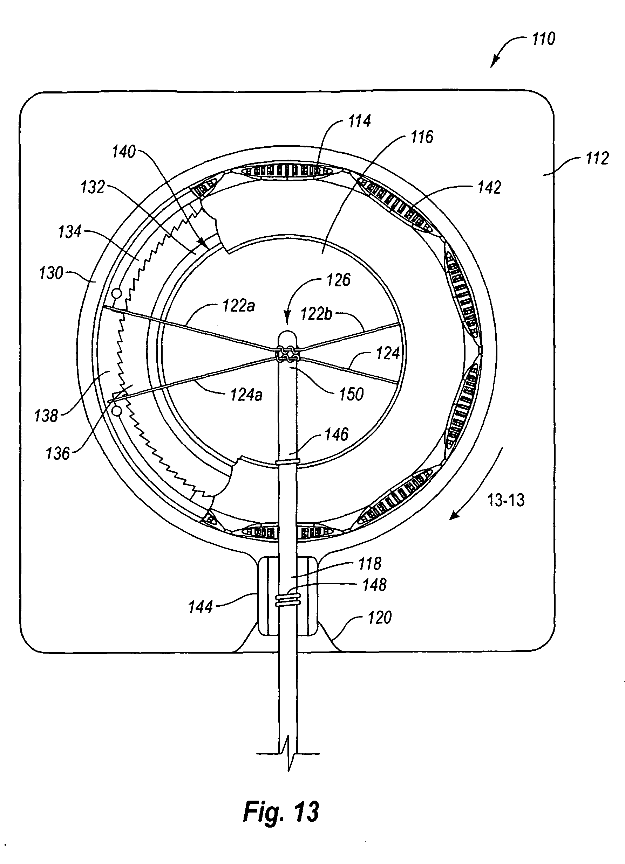

Figure 13 is a partial cross-sectional top view of an anchor device 110 illustrating anchor device 110 securing a catheter 118 to a patient according to one embodiment of the present invention. Anchor device 110 is adapted to be used with the catheter 118 that has been inserted into a patient. Anchor device 110 minimizes movement of catheter 118 that could result in pressure, discomfort, displacement, or tearing at a catheter insertion site 126. Anchor device 110 is configured to automatically deploy one or more sutures to quickly and efficiently secure the catheter 118.

In the illustrated embodiment, anchor device 110 includes an adhesive sheet 112, a rotatable ring 114, a center aperture 116, a first suture 120, a second suture 122, and a third suture 124. Adhesive sheet 112 is configured to be secured to the skin of the patient proximate catheter insertion site 126. Adhesive sheet 112 provides effective and flexible securement of anchor device 110 to the patient without causing discomfort or injury to the patient. Center aperture 116 surrounds the catheter insertion site 126. Center aperture 116 allows a user to see and access catheter insertion site 126. Center aperture 116 permits the practitioner to access catheter insertion site 126 without manipulating or removing adhesive sheet 112.

Rotatable ring 114 is coupled to adhesive sheet 112 circumscribing center aperture 116. Rotatable ring 114 is configured to be actuated by a practitioner to automatically secure the catheter 118 to minimize inadvertent or unintentional movement of catheter 118. First suture 120, second suture 122, and third suture 124 are utilized in connection with rotatable ring 114. At least one of first suture 120, second suture 122, and third suture 124 are configured to be deployed by actuation of rotatable ring 114 to automatically secure catheter 118. As the user rotates rotatable ring 114, at least one of sutures 120, 122, and 124 are deployed to automatically secure catheter 118. In the illustrated embodiment, a user rotates rotatable ring 114 in the direction of directional arrows 1-1 (clockwise direction) to deploy at least one of first suture 120, second suture 122, and third suture 124. In the illustrated embodiment, first suture 120, second suture 122, and third suture 124 have been deployed.

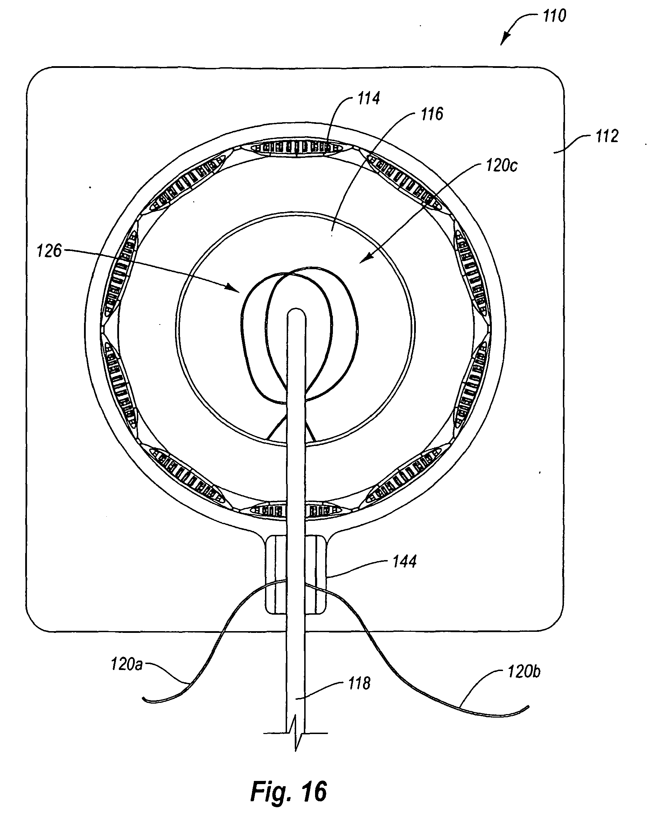

First suture 120 secures catheter 118 at first securement point 146 and second securement point 148. Second suture 122 and third suture 124 secure catheter 118 at third securement point 150. An extension saddle 144 is provided at the bottom of Rotatable ring 114. Both ends of first suture 120 are threaded to extension saddle 144. When the user pulls both ends of suture 120 to actuate first suture 120, first suture 120 automatically secures catheter 118 at first securement point 146. Subsequent to deployment of first suture 120, a user ties suture 120 at second securement point 148. Deployment of first suture 120 will be discussed in greater detail in Figure 16. Extension saddle 144 provides a desired amount of displacement between first securement point 146 and second securement point 148. The amount of displacement between first securement point 146 and second securement point 148 minimizes twisting of catheter tube 118 that could otherwise result in injury at the catheter insertion site.

In the illustrated embodiment, second suture 122 and third suture 124 provide two points of securement lateral to third securement point 150. As a result, movement of catheter 118 in a lateral direction or in a forward or rearward direction is prevented.
In the event that catheter 118 is inadvertently pulled in a rearward direction second suture 122 and third suture 124 inhibits movement of catheter 118. Similarly, in the event that catheter 118 is bumped or otherwise manipulated in a lateral direction, the two points of securement on each lateral side of third securement point 150 minimize lateral movement, twisting, or other manipulation of catheter 118 at catheter insertion site 26. As a result, discomfort, tearing, displacement, or injury at the catheter insertion site 26 resulting from inadvertent movement of catheter 118 is minimized.

In the illustrated embodiment, rotatable ring 114 includes a ratchet mechanism and a bearing member. Rotatable ring 114 comprises a rotatable outer ring 130, base 132, a bearing member 134, a ratchet ring 136, a rotatable ratchet member 138, and a suture storage channel 140. Base 132 is stationarily secured to adhesive sheet 112. Base 132 provides an implement for supporting the other components of rotatable ring 114. Rotatable ring 130 is positioned adjacent to and circumscribing base 132. Rotatable ring 130 is configured to be rotated by the user relative to base 132 to deploy second suture 122 and third suture 124.

Bearing member 134 is positioned between base 132 and rotatable outer ring 130. Bearing member 134 has bearing properties to minimize the friction between base 132 and rotatable outer ring 130 to facilitate rotation of rotatable outer ring 130 relative to base 132. In the illustrated embodiment, bearing member 134 comprises solid surface bearing material providing sliding surface bearing properties. In one embodiment, bearing member 134 comprises one or more of acetyl, high molecular weight polyethylene, lubricous plastic, Delria ®, Nylon filled with lubricant, other solid surface member, polymer material, and/or antifriction material. In the illustrated embodiment, bearing member 134 is configured to be coupled to rotatable outer ring 130 such that bearing member 134 rotates with rotatable outer ring 130. The ends of second suture 122 and third suture 124 are configured to be secured to bearing member 134. When rotatable outer ring 130 is rotated, bearing member 134 moves about the circumference of base 132 and sutures 122 and 124 are foreshortened. As a result, sutures 122 and 124 are deployed.

Ratchet ring 136 is secured to base 132 such that ratchet ring 136 is stationary relative to the adhesive sheet. Ratchet ring 136 includes a plurality of teeth or ramp portions which are configured to interact to maintain the rotational position of rotatable outer ring 130. Rotatable ratchet member 138 is coupled to rotatable outer ring 130. Rotatable ratchet member 138 includes a plurality of teeth which cooperatively interact with the teeth of ratchet ring 136. The cooperative interaction between rotatable ratchet member 138 and ratchet ring 136 allow movement of rotatable outer ring 130 in only the direction of directional arrows 13-13. As a result, rotatable ratchet member 138 and ratchet ring 136 prevent rotation of rotatable outer ring 130 in a counter clockwise direction securing the rotational position of rotatable outer ring 130. As a result, the inadvertent movement of rotatable outer ring 130 that could result in loosening of sutures 122 and 124 is prevented. In the illustrated embodiment rotatable ratchet member 138 and ratchet ring 136 comprise a ratchet mechanism.

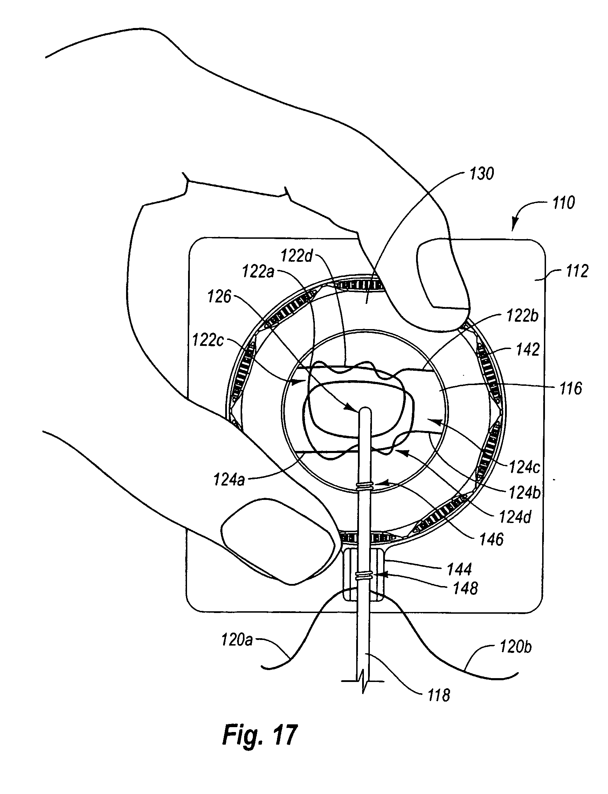

In the illustrated embodiment, suture storage channel 140 is provided on the inside diameter of base 132. Sutures 120, 122, and 124 are configured to be positioned in suture storage channel 140 previous to actuation of rotatable ring 114. As a result, previous to deployment, sutures 120, 122, and 124 are safely secured in suture storage channel 140 such that sutures 120, 122, and 124 are completely contained. When anchor device 110 is being placed on the patient, sutures 120, 122, and 124 are safely contained in the suture storage channel 140 preventing tangling of sutures 120, 122, and 124 or other interference with aspects of the procedure being performed. As the user begins to retract the ends of suture 120 in a rearward direction, suture 120 is deployed from suture storage channel 140. As the user actuates rotatable ring 114 by rotating rotatable outer ring 130, sutures 122 and 124 are deployed from their positioning in sutures storage channel 140. In one embodiment, a plurality of adhesive layers are provided between the sutures to maintain and secure the position of the sutures in the suture storage channel before deployment of the sutures. Deployment of sutures 120, 122, and 124 will be discussed in more detail in Figures 16 and 17.

In the illustrated embodiment, rotatable outer ring 130 includes a plurality of scallops 142. Scallops 142 provide variability in the relief of the upper surface of rotatable outer ring 130. As a result, the user can more easily grasp the rotatable outer ring 130 for actuation of rotatable ring 114 and actuation of sutures 122 and 124. Scallops 142 comprise slight concavities on the surface of rotatable outer ring 130. As a result, scallops do not snag articles and materials that come in contact with rotatable outer ring 130. This minimizes interference with the procedure being performed while minimizing inadvertent interruption with anchor device 110 during use. As will be appreciated by those skilled in the art, a variety of types and configurations of gripping members or anti-friction mechanisms can be utilized with rotatable outer ring 130 without departing from the scope and spirit of the present invention.

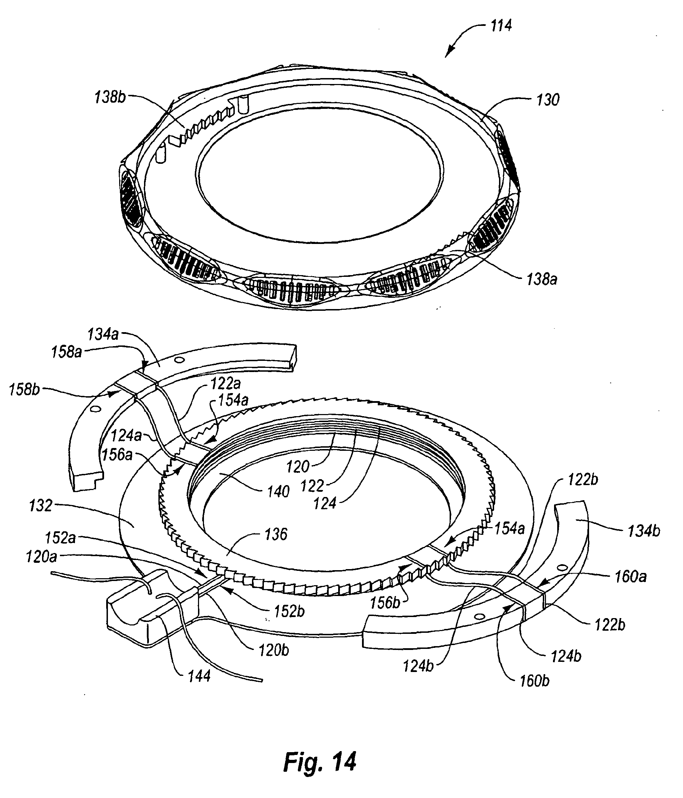

Figure 14 is an exploded view of rotatable ring 114 of anchor device 110 depicting a ratchet mechanism. In the illustrated embodiment, rotatable outer ring 130 and bearing members 134a, b are shown separated from base 132. Rotatable ratchet members 138a, b are integrally coupled to rotatable outer ring 130. A pair of pin members are positioned beneath each of rotatable ratchet members 138a, b. The pin members are configured to be positioned in bearing members 134a, b to secure bearing members 134a, b and to rotatable outer ring 130.

Bearing members 134a, b are configured to be positioned between rotatable outer ring 130 and base 132. Bearing members 134a, b contact base 132 beneath ratchet ring 136 such that bearing members 134a, b do no contact the teeth of ratchet ring 136. Similarly, bearing members 134a, b contact rotatable outer ring 130 beneath rotatable ratchet members 138a, b such that bearing members 134a, b do not contact the teeth of rotatable ratchet members 138a, b. As a result, bearing members 134a, b do not interfere with the cooperative engagement between ratchet members 138a, b and ratchet ring 136.

A lip on each of bearing members 134a, b extends inwardly beneath ratchet ring 136. When rotatable outer ring 130 is secured to bearing members 134a, b, the lateral positioning of bearing members 134a, b secures both bearing members 134a, b and rotatable outer ring 130 to base 132. Additionally, the positioning of bearing members 134a, b secures ratchet members 138a, b in cooperative engagement with ratchet ring 136.

As will be appreciated by those skilled in the art, a variety of types and configurations of bearing members can be utilized without departing from the scope of the present invention. For example, in one embodiment a circular bearing member is utilized in place of two bearing member segments. In another embodiment, one or more bearing members are integrally coupled to the rotatable outer ring. In yet another embodiment one or more bearing members are integrally coupled to the ratchet members. In yet another embodiment, a liquid bearing mechanism is utilized. In another embodiment, a roller bearing mechanism is utilized.

In the illustrated embodiment, the path of sutures 120, 122, and 124 relative to sutures storage channel 140 previous to deployment is depicted. Sutures 120, 122 and 124 are looped such that they are positioned inside suture storage channel 140. As a result, when the practitioner secures the anchor device to the patient, the practitioner does not need to manage the positioning of sutures 120, 122, and 124. Suture storage channel 140 also maintains the particular desired loop formation of sutures 120, 122, and 124 ensuring proper operation and/or deployment of sutures 120, 122, and 124.

In the illustrated embodiment, first suture channels 152a, b are positioned through base 132 and exiting at extension saddle 144. First suture 120 is configured to be positioned through first suture channels 152a, b such that the ends of first suture 120 extend from extension saddle 144. The extension of the ends of first suture 120 from the extension saddle 144 allows a user to grasp the ends of first suture 120 to actuate first suture 120.

Second suture channels 154a, b and third suture channels 156a, b are positioned through ratchet ring 136 and base 132. Bearing member suture channels 158a, b and 160a, b are positioned through bearing members 134a, b. The second suture 122 and third suture 124 are threaded through suture channels 154a, b; 156a, b; 158a, b; and 160a, b. In more particular, first end 122a of second suture 122 is threaded through second suture channel 54a and bearing member channel 58a, and is secured at the exterior of bearing member 134a. Second end 122b of second suture 122 is threaded through second suture channel 154b, through bearing member channel 160a, and is secured at the exterior of bearing member 134b. First end 124a of third suture 124 is threaded through third suture channel 156a, through bearing member channel 158b and is secured at the exterior of bearing member 134a. Second end 124b of third suture 124 is threaded through third suture channel 156b, through bearing member channel 160b, and is secured at the exterior of bearing member 134b.

Base 132 and ratchet ring 134 are stationary relative to the rotatable outer ring 130. As a result, second suture channel 154a, b and third suture channel 156a, b remain stationary during operation of rotatable ring 114. Bearing members 134a, b rotate in connection with rotatable outer ring 130. As a result, bearing member suture channels 158a, b and 160a, b rotate in connection with rotation of rotatable outer ring 130 and bearing member 134a, b. When bearing members 134a, b rotate in a clockwise direction, the ends of sutures 122 and 124 are drawn around the outside diameter of base 132 beneath ratchet ring 136. As a result, the length of first and second sutures 122 and 124 inside suture storage channel 140 is shortened. As the length of first and second sutures 122 and 124 inside suture storage channel 140 is shortened, the loops of sutures 122 and 124 become smaller such that they can no longer fit in suture storage channel 140. This results in automatic deployment of the loops of sutures 122 and 124 from suture storage channel 140.

As will be appreciated by those skilled in the art, a variety of types and configurations of sutures and suture storage channels can be utilized without departing from the scope of the present invention. For example, in one embodiment both ends of each suture exit through suture channels in the same sidewall of the base of the rotatable ring. In another embodiment, the suture channels comprise bores rather than slots. In another embodiment, the ends of the suture are integrally affixed to the bearing members. In yet another embodiment, four independent sutures are utilized in the place of the first and second suture. In the illustrated embodiment, the suture storage channel is shown without a retention flange for the sake of clarity.

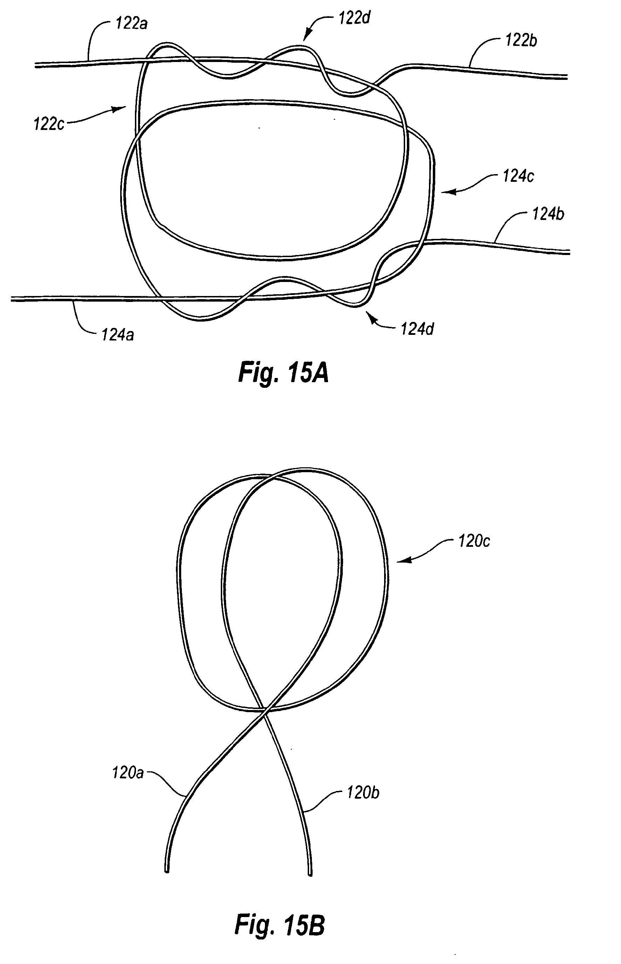

Figure 15 illustrates the loop configuration of sutures 120, 122, and 124 utilized to secure a catheter. In the illustrated embodiment, first suture 120 includes a first end 120a, a second end 120b, and a double loop portion 120c. Double loop portion 120c of first suture 120 is configured to form a clove hitch when secured about a catheter. Previous to deployment from the suture storage channel 140 (see Figure 14) the double loop configuration of double loop portion 120c is adapted to be wide enough to fit within the suture storage channel 140 depicted in Figure 14.

In the illustrated embodiment, second suture 122 includes a first end 122a, a second end 122b, a loop portion 122c, and a double wrapped portion 122d. As previously discussed, first end 122a is configured to be threaded through a plurality of suture channels and be secured adjacent a bearing member. Similarly second end 122b is configured to be threaded through a plurality of suture channels and be secured adjacent a bearing member. Previous to deployment, loop portion 122c is configured to fit easily within the stuture storage channel 140 depicted in Figure 14. Subsequent to deployment, loop portion 122c is tightened around the catheter. The double wrapped portion 122d is configured to maintain the tightened configuration of loop portion 122c subsequent to deployment of second suture 122.

In the illustrated embodiment, third suture 124 includes a first end 124a, a second end 124b, a loop portion 124c, and a double wrapped portion 124d. As previously discussed, first end 124a is configured to be threaded through a plurality of suture channels and be secured adjacent a bearing member. Similarly second end 24b is configured to be threaded through a plurality of suture channels and be secured adjacent a bearing member. Previous to deployment, loop portion 124c is configured to fit easily within the stuture storage channel 140 depicted in Figure 14. Subsequent to deployment, loop portion 124c is tightened around the catheter. The double wrapped portion 124d is configured to maintain the tightened configuration of loop portion 124c subsequent to deployment of third suture 124.

The loop portions 122c and 124c of second suture 122 and third suture 124 are adapted to overlap with one another. The overlapping configuration of second suture 122 and third suture 124 is configured to provide cooperative securement of a catheter subsequent to deployment of second suture 122 and third suture 124. As will be appreciated by those skilled in the art, a variety of types and configurations of sutures and suture loops can be utilized without departing from the scope and spirit of the present invention. For example, in one embodiment both ends of second suture are positioned on one lateral side while both ends of third suture are positioned on the opposite lateral side. In another embodiment, the loops of second suture and third suture are coupled to one another such that the sutures are centered during closure of the loop portions.

Figure 16 is a top view of the anchor device 110 illustrating deployment of first suture 120. To deploy first suture 120 a user grasps first end 120a and second end 120b and retracts in a rearward direction. The double loop portion 120c of first suture 120 is deployed as first end 120a and second end 120b are pulled in a rearward direction. Tensioning occurs as first end 120a and second end 120b are retracted further in the distal direction. In the illustrated embodiment, both the first loop and second loop of the double loop portion 120c have been deployed but have not substantially tightened around catheter 122. The first loop and second loop of double loop portion 120c are configured to encircle catheter 118.

In the illustrated embodiment, the double loop clove-hitch configuration of first suture 120 secures catheter 118 to rotatable ring 114 at a first securement point 146 (see Figure 13). By maintaining the tension on first end 120a and second end 120b, first suture 120 retains catheter 118 against rotatable ring 114. Once first suture 120 is tightened about catheter 118 at first securement point 146 (see Figure 13), first end 120a and second end 120b are tied to catheter 118 at second securement point 148 at extension saddle 144 (see Figure 13).

As will be appreciated by those skilled in the art, the time required to secure catheter 118 utilizing first suture 120 can be as little as a few seconds. Not only is time saved in comparison to conventional techniques, but the steps of securing catheter 118 utilizing first suture 120 is conceptually simple, and readily conducted by a surgeon, by an assisting nurse, or other practitioner.

As will be appreciated by those skilled in the art, a variety of types and configurations of mechanisms for securing the catheter can be utilized without departing from the scope of the present invention. In one embodiment, the securement of the catheter utilizes a triple loop clove-hitch configuration. In another embodiment, the securement of catheter utilizes two double loop clove-hitch configurations from a first and second suture. In another embodiment, the suture secures the catheter utilizing an arrangement other than a clove-hitch loop. In yet another embodiment, the anchor device includes two or more resilient pockets from which multiple sutures can be deployed to secure the catheter against the rotatable ring 114.

Figure 17 depicts deployment of second suture 122 and third suture 124 to secure catheter 118 proximate catheter insertion site 126. To actuate sutures 122 and 124, the practitioner rotates rotatable outer ring 130 of rotatable ring 114. In the illustrated embodiment, rotatable ring 114 includes a rotatable outer portion of the ring and a stationary base 132. The practitioner rotates rotatable ring 114 in the clockwise direction, for example, approximately 1/8^{th} of a turn or 45 degrees. This rotation causes a portion of second suture 122 and third suture 124 to deploy from within suture storage channel 140.

Rotation of rotatable outer ring 130 results in movement of both bearing members (not shown). Because the one end of both second suture 122 and second suture 124 are secured to a bearing member (not shown) on both sides of rotatable ring 114, rotation of the rotatable outer ring 130 pulls the ends of both sutures around the outer circumference the base of rotatable ring 114. As a result, ends 122a and 122b of second suture 122 are pulled in opposite directions. Similarly, ends 124a and 124b of third sutures 124 are pulled in opposite directions. As a result, loop portions 122c and 124c are tightened without exerting undue pressure on catheter 118 or catheter insertion site 126.

As will be appreciated by those skilled in the art, a variety of types and configurations of mechanisms can be utilized in connection with deployment of the second and third sutures according to the present invention. For example, in one embodiment a slidable engagement mechanism such as a clip, ring, or preformed loop is utilized to form the loops of the first and second sutures. In another embodiment, a single suture is utilized in place of the second and third sutures. In yet another embodiment, three or more sutures are deployed during rotation of the rotatable ring.

As will be appreciated by those skilled in the art, the amount of time required to deploy second and third sutures 122 and 124 by actuating rotatable ring 114 is mere seconds. Additionally, as second and third sutures 122 and 124 are tightened about catheter 118 (as depicted in Figure 13), the ratchet mechanism maintains securement of second and third sutures 122 and 124 about catheter 118. The ratchet mechanism also allows the user to tighten sutures 122 and 124 subsequent to any natural loosening of the suture thread.

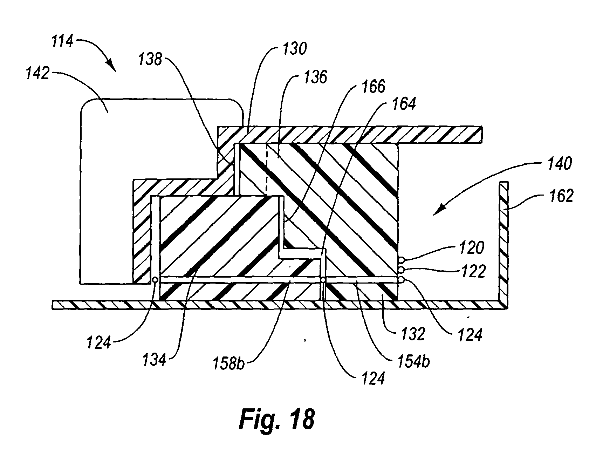

Figure 18 is a cross-sectional side view of a portion of the rotatable ring 114 illustrating a bearing member 134 of the rotatable ring 114. In the illustrated embodiment, bearing member 134 is positioned between rotatable outer ring 130 and base 132. Base 132 includes a horizontal base member and an upright member. Ratchet ring 136 is integrally coupled to the upright member of base 132. Bearing member 134 is positioned on top of the horizontal member of base 132 and adjacent the upright member of base 132. Additionally, the top surface of bearing member 134 is positioned beneath ratchet ring 136. Bearing member 134 contacts the upright member of base 132 at interface 166. The profile of bearing member 134 and the upright member of base 132 results in the formation of slot 164. Slot 164 is configured to accommodate a suture (suture 124 in the illustrated embodiment) as bearing 134 moves around the circumference of base 132.

Rotatable outer ring 130 is positioned above and on the outside diameter of rotatable ring 114. Rotatable outer ring 130 is the portion of rotatable ring 114 that is grasped by the user to actuate rotatable ring 114. In the illustrated embodiment, the bottom surface of rotatable outer ring 130 is secured to bearing member 134. Rotatable ratchet member 138 is positioned above bearing member 134 and in contact with ratchet ring 136. As the user rotates rotatable outer ring 130, the teeth of rotatable ratchet member 138 slip in a counter clockwise direction past the teeth of ratchet ring 136. The teeth of ratchet ring 136 and ratchet member 138 cooperatively interact to prevent movement of the teeth of ratchet member 138, and thus rotatable outer ring 130, in a counterclockwise direction.

In the illustrated embodiment, sutures 120, 122, and 124 are depicted in suture storage channel 140. A flange 162 is secured to base 132. Flange 162 is positioned laterally inward from sutures 120, 122, and 124. Flange 162 maintains the position of sutures 120, 122, and 124 in suture storage channel 140 until deployment of sutures 120, 122, and 124. As sutures 120, 122, and 124 deploy and the loop portions of sutures 120, 122, and 124 become smaller, sufficient force is exerted on flange 162 to bend flange 162 inwardly. Additionally, as sutures 120, 122, and 124 deploy, flange 162 pushes sutures 120, 122, and 124 in an upward direction while bending inwardly.
The upward movement of sutures 120, 122, and 124 facilitates the positioning of sutures 122 and 124 around catheter 118 (see Figure 13) at a desired degree of displacement from the patient's skin. This facilitates cleaning of the catheter insertion site without interference of sutures 122 and 124.

As will be appreciated by those skilled in the art, a variety of types and configurations of rotatable rings and bearing members can be utilized without departing from the scope of the present invention. For example, the configuration and number of contact surfaces between the bearing members and rotatable rings can vary. In another embodiment, the bearing member includes ratchet surfaces that interact with one or more surfaces of the base and/or rotatable outer ring.

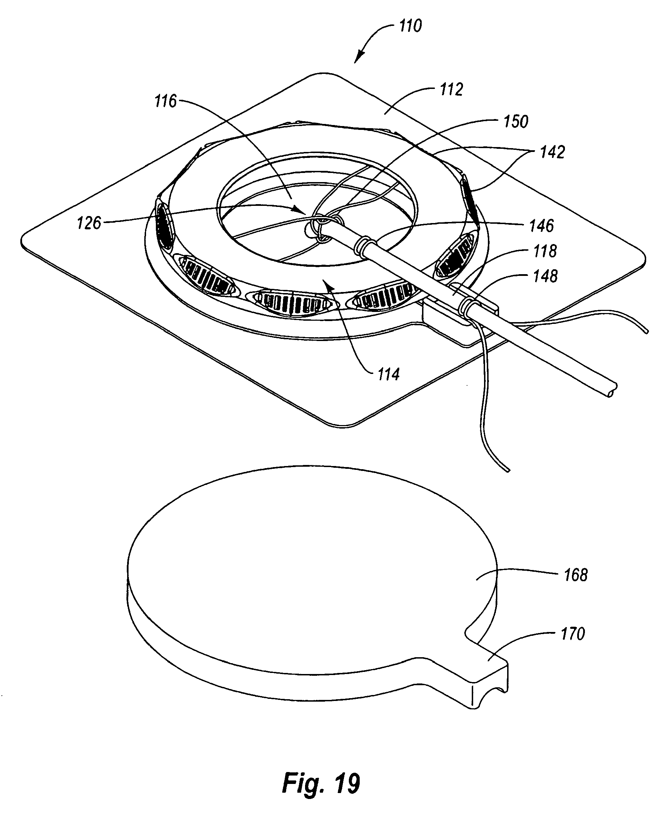

Figure 19 is a perspective view of the anchor device 110 of Figure 13 and a removable lid 168 for covering center aperture 116 and catheter insertion site 126. In the illustrated embodiment, removable lid 168 is configured to provide protection to the catheter insertion site 126 from environmental interference. For example, in the event that a patient desires to take a shower, removable lid 168 can be placed over rotatable ring 114 to prevent the inflow of water or inadvertent injury to the catheter insertion site 126. This allows for continued use and securement of the catheter 118 while minimizing the potential for injury or interference with proper operation of the catheter insertion site 126.

In the illustrated embodiment, the rim of the removable lid 168 is configured to fit over the rotatable ring 114 to secure the removable lid 168 relative to the anchor device 110. An extension member 170 having an inner channel is provided to permit passage of catheter 118 to the exterior of the lid while permitting sealing and protection of center aperture 116.

As will be appreciated by those skilled in the art, a variety of types and configurations of removable lids can be utilized without departing from the scope of the present invention. For example, in one embodiment, the removable lid can be configured to be utilized the majority of the usage time of the anchor device. Alternatively, the use of the removable lid can be limited to the occasional situation when extra care or caution is deemed necessary to prevent injury to the catheter insertion site. In one embodiment, the removable lid is clear to permit observation of the catheter insertion site when the removable lid is positioned over the rotatable ring. In another embodiment, the removable lid is secured to the anchor device with a lanyard or other securement device when the removable lid is not in operation. This assures that the removable lid will be readily accessible in the event the practitioner needs to utilize the removable lid during usage of the anchor device.

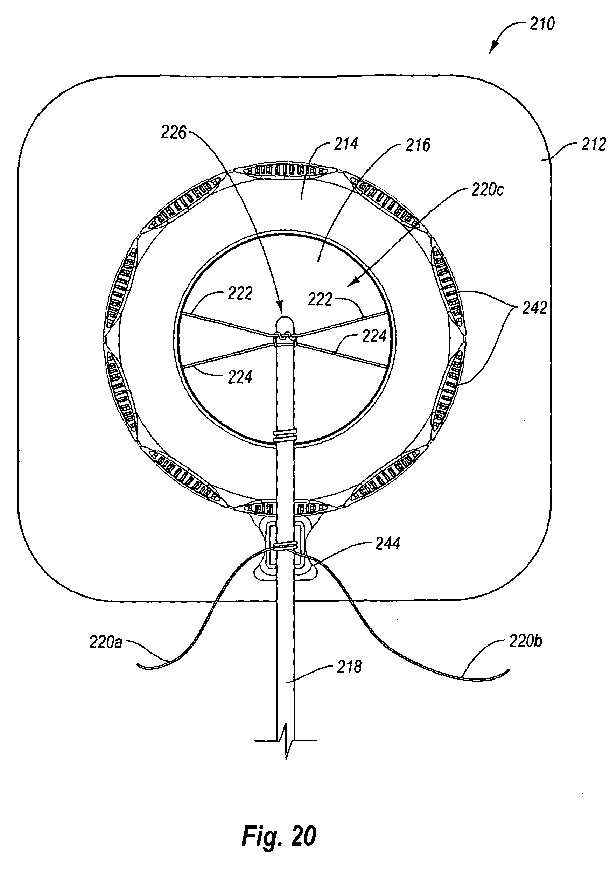

Figure 20 is a top perspective view of anchor device 210 subsequent to deployment of rotatable ring 214. In the illustrated embodiment, catheter 218 is secured by first suture 220, second suture 222, and third suture 224, subsequent to deployment of rotatable ring 214 and first suture 220. First suture 220 secures catheter 218 adjacent the bottom of rotatable ring 214 and at extension saddle 244. Extension saddle 244 provides a desired degree of displacement between the points of securement provided by suture 220 relative to catheter 218. The displacement provided between the points of securement of first suture 220 is sufficient to substantially minimize kinking, twisting, or other manipulation of catheter 218 that could result in damage to the patient tissue at catheter insertion site 226 resulting from movement of catheter 218. Additionally, extension saddle 244 provides a groove which accommodates catheter 218 to minimize kinking, pinching, or other pressure on catheter 218 from the transition over the top of rotatable ring 214.

The portion of catheter 218 positioned adjacent catheter insertion site 226 is secured by second suture 222 and third suture 224. One portion of second suture 222 is positioned on the left side of rotatable ring 214, while a second portion of second suture 222 is secured adjacent the right side of rotatable ring 214. One portion of third suture 224 is secured adjacent the left side of rotatable ring 214, while the second portion of second suture 222 is secured adjacent the right side of rotatable ring 214. Additionally, the first portions of second suture 222 and third suture 224 on the left side of rotatable ring 214 are positioned at a displacement between five and 65 degrees or more relative to one another to provide securement of catheter 218 to minimize forward and rearward movement of catheter 218 during use of the anchor device 210. Additionally, the second portions of second suture 222 and third suture 224 on the right side of rotatable ring 214 are positioned at an angle of between five and 65 degrees or more relative to one another to minimize forward and rearward movement of the catheter 218 which could result in injury at catheter insertion site 226. As a result, a total of four separate points of securement are provided at the portion of catheter 218 adjacent catheter insertion site 226 to minimize both lateral and forward and rearward movement of catheter 218 during usage of catheter anchor device 210. This provides a safe and reliable securement of catheter 218 during usage while also providing access to the catheter insertion site for cleaning and care of the catheter and/or patient tissue at the catheter insertion site 226. In the illustrated embodiment, a plurality of scallops 242 are shown on rotatable ring 214. Scallops 242 facilitate gripping of rotatable ring during actuation of rotatable ring. Scallops 242 comprise a concave depression in the outward surface of rotatable ring 214. By providing a concave depression in the outward surface of rotatable ring 214, scallops 242 provide gripping members which minimize any potential abrasion to the patient or practitioner utilizing anchor device 210.

The configuration of anchor device 210 and rotatable ring 214 allow for quick, simple, and effective securement of catheter 218 subsequent to placement of catheter 218 in a patient. This not only shortens the length of the catheter securement procedure and thus the entire catheter placement procedure, but also is sufficiently simple such that an assisting nurse or other caretaker can secure catheter 218 while the physician attends to other aspects of the procedure being performed. This is not only more efficient from the standpoint of operating room economics, but can also be quite helpful in time sensitive procedures such as in a trauma setting or emergency situation.

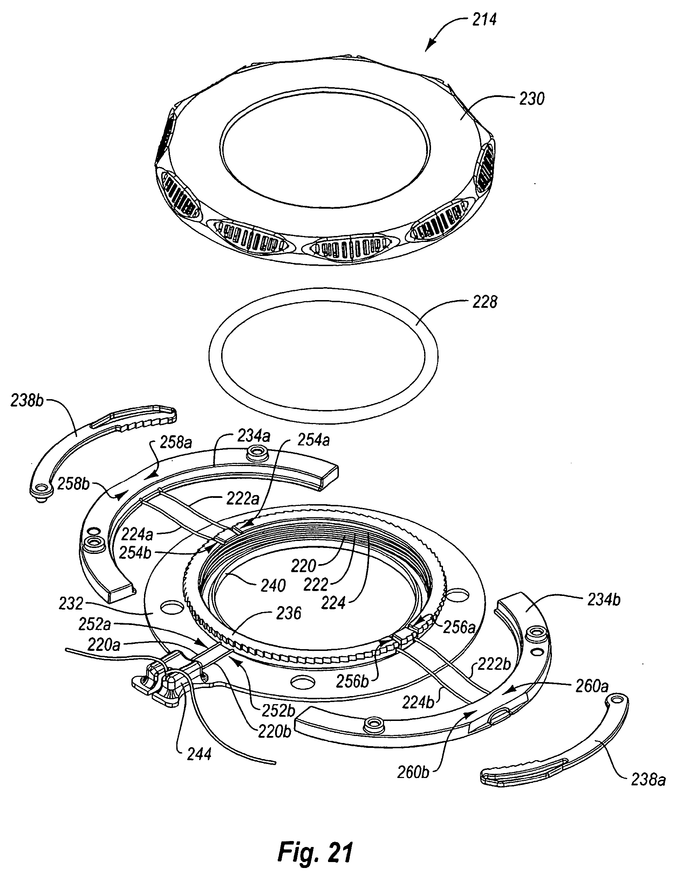

Figure 21 is an exploded view of rotatable ring 214 of anchor device 210 depicting a ratchet mechanism. In the illustrated embodiment, rotatable outer ring 230 and bearing members 234a, b are shown separated from base 232. A plurality of pin members are positioned beneath rotatable outer rings. The pin members are configured to be positioned in bearing members 234a, b to secure bearing members 234a, b to rotatable outer ring 230.

Bearing members 234a, b are configured to be positioned between rotatable outer ring 230 and base 232. Bearing members 234a, b contact base 232 beneath ratchet ring 236 such that bearing members 234a, b do no contact the teeth of ratchet ring 236. Similarly, bearing members 234a, b contact rotatable outer ring 230 beneath rotatable ratchet members 238a, b such that bearing members 234a, b do not contact the teeth of rotatable ratchet members 238a, b. As a result, bearing members 234a, b do not interfere with the cooperative engagement between ratchet members 238a, b and ratchet ring 236.

A lip on each of bearing members 234a, b extend inwardly beneath ratchet ring 236. When rotatable outer ring 230 is secured to bearing members 234a, b, the lateral positioning of bearing members 234a, b secures both bearing members 234a, b and rotatable outer ring 230 to base 232. Additionally, the positioning of bearing members 234a, b maintains ratchet members 238a, b in cooperative engagement with ratchet ring 236. In the illustrated embodiment, bearing members 234a, b include a securement member for securing the sutures 222 and 224 during rotation of bearing members 234a, b.

As will be appreciated by those skilled in the art, a variety of types and configurations of bearing members can be utilized without departing from the scope of the present invention. For example, in one embodiment a circular bearing member is utilized in place of two bearing member segments. In another embodiment, one or more bearing members are integrally coupled to the rotatable outer ring. In yet another embodiment one or more bearing members are integrally coupled to the ratchet members. In another embodiment, the bearing members are positioned above the ratchet ring. In yet another embodiment, a liquid bearing mechanism is utilized. In another embodiment, a roller bearing mechanism is utilized.

In the illustrated embodiment, the path of sutures 220, 222, and 224 relative to sutures storage channel 240 previous to deployment is depicted. Sutures 220, 222 and 224 are looped such that they are positioned inside suture storage channel 240. As a result, when the practitioner secures the anchor device to the patient, the practitioner does not need to manage the positioning of sutures 220, 222, and 224. Suture storage channel 240 in combination with O-ring 228 also maintains the particular desired loop formation of sutures 220, 222, and 224 ensuring proper operation and/or deployment of sutures 220, 222, and 224.

In the illustrated embodiment, first suture channels 252a, b are positioned through base 232 and exiting at extension saddle 244. First suture 220 is configured to be positioned through first suture channels 252a, b such that the ends of first suture 220 extend from extension saddle 244. The extension of the ends of first suture 220 from the extension saddle 244 allows a user to grasp the ends of first suture 220 to actuate first suture 220.

Second suture channels 2254a, b and third suture channels 2256a, b are positioned through ratchet ring 236 and base 232. Bearing member suture channels 2258a, b and 260a, b are positioned through bearing members 234a, b. The second suture 222 and third suture 224 are threaded through suture channels 2254a, b; 256a, b; 258a, b; and 260a, b. In more particular, first end 22a of second suture 222 is threaded through second suture channel 254a and bearing member channel 258a, and is secured at the exterior of bearing member 234a. Second end 222b of second suture 222 is threaded through second suture channel 254b, through bearing member channel 260a, and is secured at the exterior of bearing member 234b. First end 224a of third suture 224 is threaded through third suture channel 256a, through bearing member channel 58b and is secured at the exterior of bearing member 234a. Second end 224b of third suture 224 is threaded through third suture channel 256b, through bearing member channel 60b, and is secured at the exterior of bearing member 234b.

Base 232 and ratchet ring 234 are stationary relative to the rotatable outer ring 230. As a result, second suture channel 254a, b and third suture channel 256a, b remain stationary during operation of rotatable ring 214. Bearing members 234a, b rotate in connection with rotatable outer ring 230. As a result, bearing member suture channels 2258a, b and 260a, b rotate in connection with rotation of rotatable outer ring 230 and bearing member 234a, b. When bearing members 234a, b rotate in a clockwise direction, the ends of sutures 222 and 224 are drawn around the outside diameter of base 232 beneath ratchet ring 236. As a result, the length of first and second sutures 222 and 224 inside suture storage channel 240 is shortened. As the length of first and second sutures 222 and 224 inside suture storage channel 240 is shortened, the loops of sutures 222 and 224 become smaller such that they can no longer fit in suture storage channel 240. This results in automatic deployment of the loops of sutures 222 and 224 from suture storage channel 240.

Rotatable ratchet members 238a, b engage the teeth of ratchet ring 236 to minimize counterclockwise movement of rotatable ring 214 that would result in loosening of sutures 222 and 224. During rotation of rotatable outer ring 230, bearing members 234a, b and rotatable ratchet members 238a, b are rotated in a clockwise direction about base 232 and in particular ratchet ring 236. Rotatable ratchet members 238a, b engage the teeth of ratchet ring 236 as rotatable ratchet members are advanced 38a, b in the clockwise direction. When a user discontinues rotation of rotatable outer ring, rotatable ratchet members 238a, b engage the teeth of ratchet ring 236 minimizing movement of rotatable outer ring in a counterclockwise direction that would otherwise loosen second suture 222 and third suture 224.

Rotatable ratchet member 238a is positioned between bearing member 234a and rotatable outer ring 230. A pivot pin is positioned on the bottom surface of each of the rotatable ratchet members 238a, b. The pivot pins are is positioned in rotation bores of the corresponding bearing members 234a,b to pivotally couple rotatable ratchet member 234a to bearing member 234a. Rotatable outer ring 230 contacts the upper surface of rotatable ratchet member 238a to maintain contact between the pivot pins and the rotation bores.

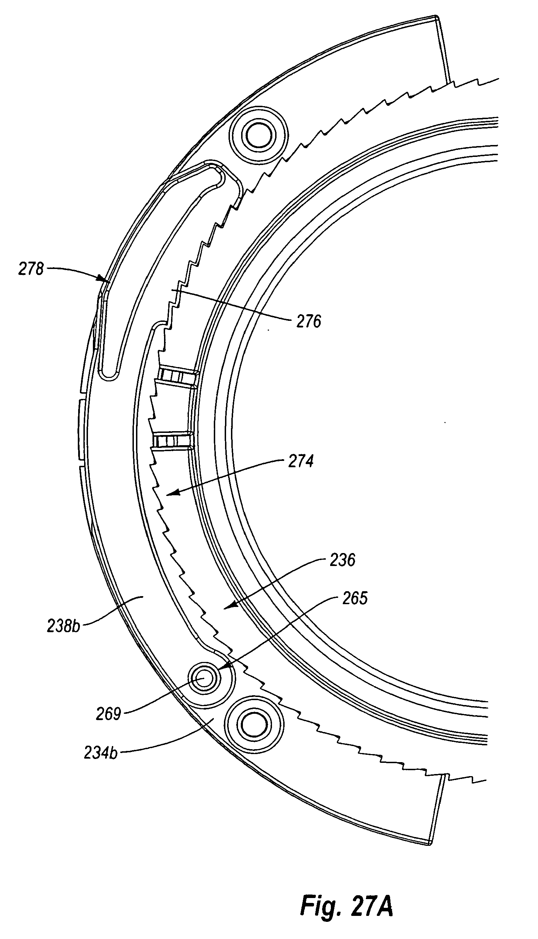

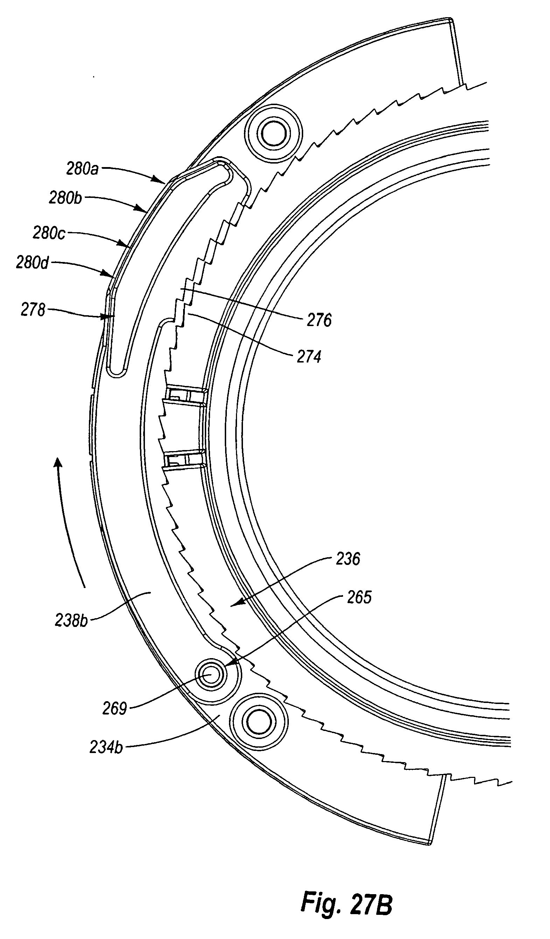

The end of rotatable ratchet members 238a, b positioned opposite the pivot point provided by the pivot pin of rotatable ratchet member 238a, b and the bore of bearing members 234a,b include a spring member and a plurality of teeth. The plurality of teeth engage the teeth of ratchet ring 236 to minimize movement of rotatable outer ring 230 and bearing members 234a,b in a counterclockwise direction. This spring is provided by the cutaway portion in the head of rotatable ratchet members 238a,b and the resilient nature from the material from which the heads of rotatable ratchet members 238a,b are constructed. Rotatable ratchet members 238a, b will be discussed in more detail with reference to Figures 27A and 27B.

In the illustrated embodiment, an O-ring 228 is also illustrated. O-ring 228 is configured to be sandwiched between rotatable outer ring 230 and base 232 to maintain the position of first suture 220, second suture 222, and third suture 224 beneath rotatable ring 214. By maintaining the position of first suture 220, second suture 222, and third suture 224, disruption of the sutures before deployment of the sutures is minimized and reliable and proper operating of anchor device 210 is maintained. As a result, O-ring 228 provides a simple and reliable mechanism for storing first suture 220, second suture 222, and third suture 224 beneath rotatable ring 214 during storage of anchor device 210, during securement of adhesive sheet 212 to the patient, or other aspects of the securement procedure performed before actuation of sutures 220, 222, and 224.

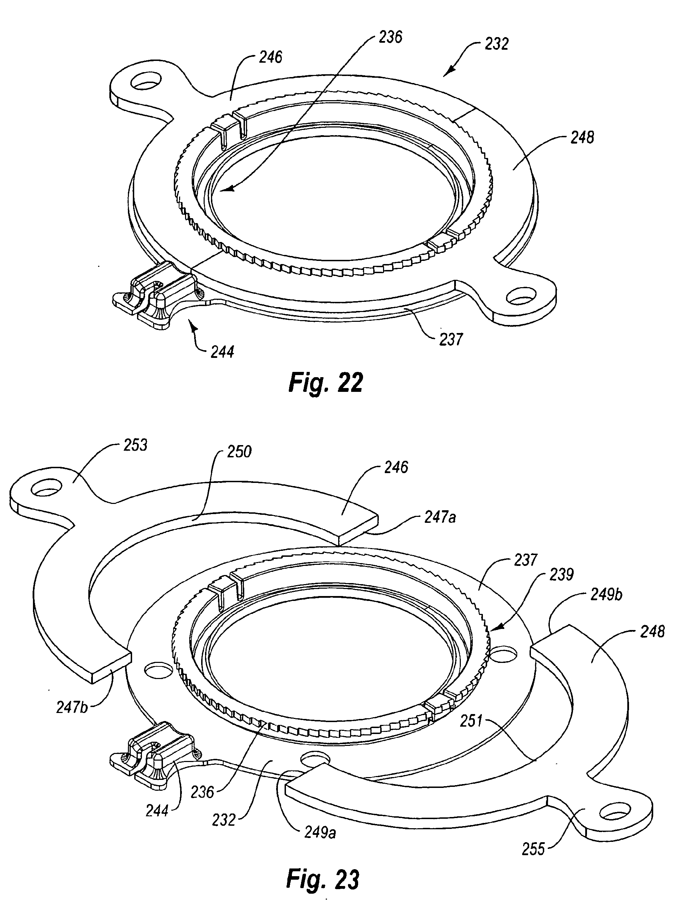

Figure 22 and 23 are perspective views of base 232 during molding of base 232. In the illustrated embodiment, base 232 comprises a single molded member formed utilizing first and second mold members 46 and 48. Base 232 comprises a ratchet ring 236 and an extension saddle 244. In the illustrated embodiment, base 232 incudes an undercut 239 positioned between ratchet ring 236 and base flange 237. Undercut 239 substantially complicates the molding of base 232. As a result, first mold member 246 and second mold member 248 are utilized to provide the undercut during the molding of base 232. For the sake of clarity, the other mold members utilized to form base 232 have not been illustrated to more clearly depict operation of first mold member 246 and second mold member 248.

In the illustrated embodiment, first mold member 246 comprises mold member interfaces 247a, b, an inner circumference 250, and a gripping handle 253. Second mold member 248 comprises mold member interfaces 249a, b, an inner circumference 251, and gripping handle 255. During molding, mold member interfaces 247a, b of first mold member contact mold member interfaces 249a, b of second mold member 248. Inner circumference 250 of first mold member 246 and inner circumference 251 of second mold member 248 form the undercut 239 positioned between ratchet ring 236 and base flange 237. Inner circumference 250 and inner circumference 251 define the inner boundary of undercut 239. The top portion of first mold member 246 and second mold member 248 define the upper lateral surface extending from the innermost horizontal surface of uppercut 239 (not shown) to the edge of ratchet ring 236. The bottom of first mold member 246 and second mold member 248 form the lower horizontal surface which extends from the inner vertical surface of undercut 239 (not shown) and extends outward to be coextensive with base flange 237.

In one embodiment, the surfaces of undercut 239 are slightly flared or tapered to allow for proper releasing of first mold member 246 and second mold member 248 such that when a user grasps gripping handles 253 and gripping handle 255 to pull first mold member 246 and second mold member 248 in a rearward direction, first mold member and second mold member 246, 248, automatically release and can easily be slid from undercut 239. In this manner, base 232 can be molded in single member ensuring continuity of surfaces and reliable and proper operation of the components of base 232 during use of anchor device 210.

As will be appreciated by those skilled in the art, a variety of types and configurations of mechanism can be utilized to mold base 232 in a unitary fashion without departing from the scope of the present invention. For example, first and second mold members which are configured to be automatically retracted by an automated molding apparatus or other machinery can be utilized. In another embodiment, a single mold member which is hinged, bendable, meltable or otherwise manipulable to remove the mold member from undercut 239 is utilized. In another embodiment, mold members having different form, size, and/or surfaces can be utilized. In another embodiment, more than two mold members are utilized to form the undercut and/or other portions of the base during molding.

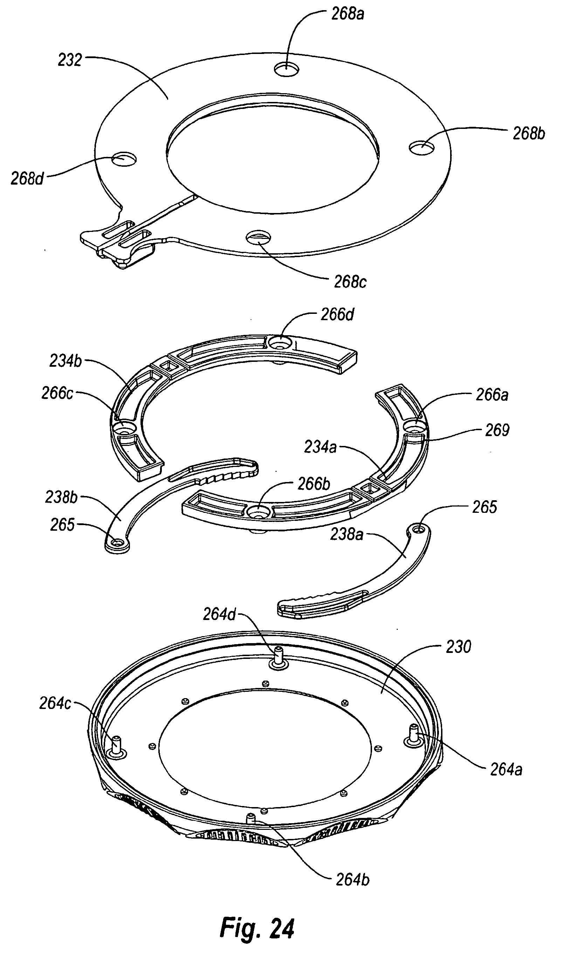

Figure 24 is a bottom exploded view illustrating the manner in which the components of anchor device 210 are secured during assembly. In the illustrated embodiment, rotatable outer ring 230, rotatable ratchet member 238a, bearing member 234a, and base 232 are depicted. Bearing member 234a is configured to be sandwiched between rotatable outer ring 230 and base 232. Rotatable ratchet member 238a is configured to be positioned between bearing member 234a and rotatable outer ring 230. Bearing member 234a is configured to be attached directly to rotatable outer ring 230 while being slidable relative to base 232. Pins 264a-c are positioned on the underside of rotatable outer ring 230 engage securement bores 266a-d of bearing member 234a, b. Contact between bearing member 234a and base 232 maintains contact between securement bores 266a-d and pins 264a-d. In the illustrated embodiment, pins 264a, b are configured to be welded to securement bores 266a, b to integrally couple bearing member 234a to rotatable outer ring 230 subsequent to assembly of rotatable outer ring and base 232. Pins 264c, d are configured to be welded to securement bores 266c, d to integrally couple bearing member 234b to rotatable outer ring 230 subsequent to assembly of rotatable outer ring and base 232. A plurality of access bores 268a-d are provided in connection with base 232 such that the welding tool can be inserted through access bores 268a-d to weld pins 264a-d to securement bores 266a-d of bearing members 234a, b.

Rotatable ratchet member 238a is positioned between bearing member 234a and rotatable outer ring 230. In the illustrated embodiment, a pivot pin is positioned on the upper surface of bearing member 234a. The pivot pin is positioned in rotation bore 265 to pivotally couple rotatable ratchet member 234a to bearing member 234a. Rotatable outer ring 230 contacts the upper surface of rotatable ratchet member 238a to maintain contact between the pivot pins and rotation bore 265. Additionally, the outer horizontal portion of the rotatable outer ring which extends downward adjacent rotatable ratchet member 238a contains lateral movement of the free end of rotatable ratchet member 238a to ensure proper operation and contact between the teeth of rotatable ratchet member 238a and the teeth of ratchet ring 236 (illustrated in Figures 22 and 23).

As will be appreciated by those skilled in the art, a variety of types and configurations of mechanisms for securing the components of the rotatable ring can be utilized without departing from the scope of the present invention. For example, in one embodiment, the bearing member is configured to be welded to the rotatable outer ring previous to assembly with the base. In another embodiment, a snap fitting is provided between the bearing member and the rotatable outer ring. In another embodiment, a continuous bearing member is integrated with the base 232 while the rotatable ratchet member is secured independently to the rotatable outer ring. In yet another embodiment, a surface is provided on the bearing member to maintain proper operation of the rotatable ratchet member.

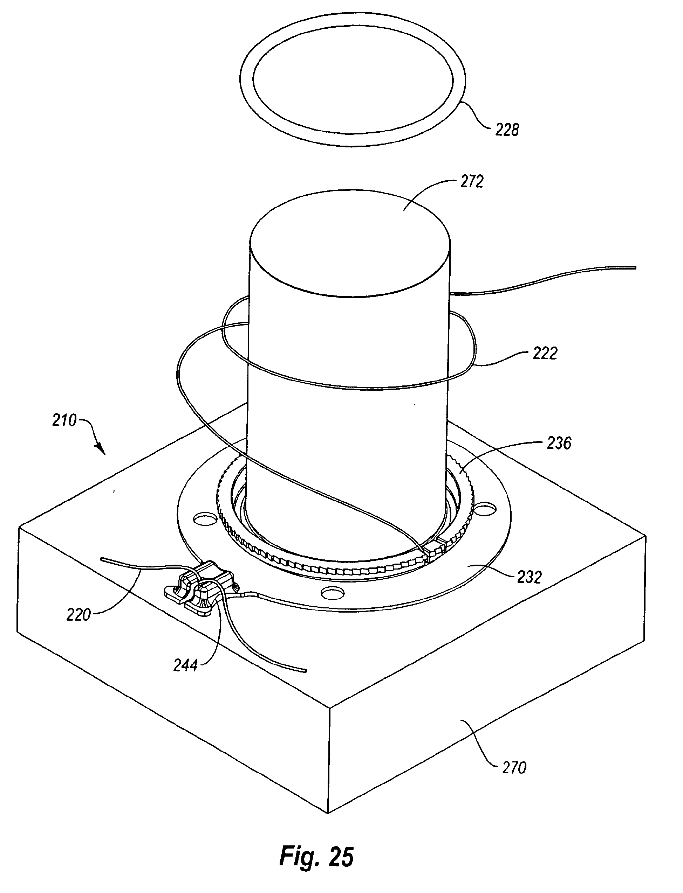

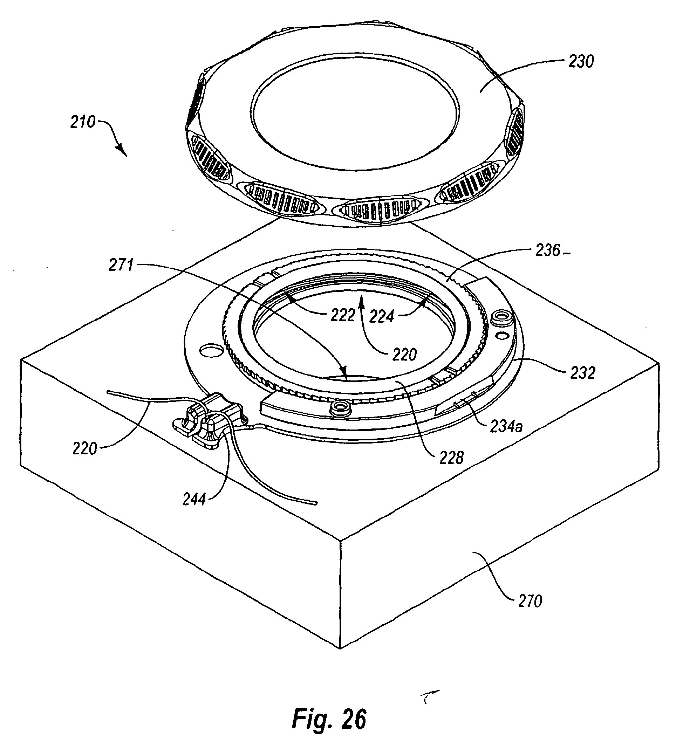

Figures 25 and 26 illustrate loading of the sutures utilized with anchor device, according to one embodiment of the present invention. In the illustrated embodiment, base 232 of anchor device 210 is mounted on a loading block 270. Center aperture 216 of anchor device 210 is positioned over the center bore 271 of loading block 270.
A suture loading cylinder 272 is positioned through center bore 271 and center aperture 216 such that the wall of the suture loading cylinder 272 is positioned adjacent the inner portion of ratchet ring 236. Suture loading cylinder 272 is utilized to provide a quick and effective mechanism for forming the loop configurations of first suture 220, second suture 222, and third suture 224 (not shown) and for loading the sutures in base 232.

In the illustrated embodiment, the loops of second suture 222 are being formed around suture loading cylinder 272. First suture 220 has previously been loaded into base 232 utilizing suture loading cylinder 272. One end of second suture 222 has been threaded through and secured relative to base 232. The loop has been substantially formed by wrapping the length of second suture 222 about suture loading cylinder 272 in a manner so as to produce the desired configuration of the loop portion of second suture 222. The second end of second suture 222 is positioned freely, as it has not yet been secured relative to base 232. Once the loop has been formed in second suture 222, the loop portion of second suture 222 will be drawn tightly around suture loading cylinder 272 by pulling on the second end of second suture 222. The second end of second suture 222 will then be threaded relative to base and secured to one of the bearing members 234a, b (bearing member 234b not shown).

As the loop portion of second suture 222 is drawn tight and the second end of second suture 222 is secured relative to one of bearing members 234a, b and base 232, the loop portion of second suture 222 is automatically drawn down such that it is loaded within ratchet ring 236 in the desired position for deployment. As will be appreciated by those skilled in the art, similar steps, acts, and processes are utilized to load first suture 220 and third suture 224. The discussion of the formation of loops of second suture 222 and the loading of second suture 222 in base 232 is discussed for illustrative purposes and should in no way be considered to be limiting in nature.

Once first suture 220, second suture 222, and third suture 224 have been properly loaded in base 232, O-ring 228 is positioned over the top of suture loading cylinder 272. O-ring 228 is lowered along the length of suture loading cylinder 272 until it is positioned along the inner circumference of ratchet ring 236 effectively maintaining the position of first suture 220, second suture 222, and third suture 224 in their desired position within base 232. Once O-ring 228 has been properly positioned within base 232, suture loading cylinder 272 is withdrawn from center aperture 216 and rotatable outer ring 230 is lowered into engagement with base 232 as discussed with respect to Figure 24. The proper steps can then be taken to couple rotatable outer ring 230 to bearing member 234a, b as discussed with respect to Figure 24.

As will be appreciated by those skilled in the art, a variety of types and configurations of mechanism for loading the sutures in the base can be utilized without departing from the scope of the present invention. For example, in one embodiment, a loading block and suture loading cylinder are utilized to manually load the sutures in the anchor device. In another embodiment, the loading block and suture loading cylinder are utilized with automated processes to load the suture into the base or other component of the anchor device.

Figures 27A and 27B illustrate rotatable ratchet member 238b in operation with respect to ratchet ring 236. While rotatable ratchet member 238b is shown for illustrative purposes, it will be understood by those skilled in the art that operation of rotatable ratchet member 238b also is exemplary of rotatable ratchet member 238a (see Figure 21.) In the illustrated embodiment, rotatable ratchet member 238b is rotatably coupled to bearing member 234b utilizing pivot pin 269 and rotation bore 265 of rotatable ratchet member 234b. Rotatable ratchet member 238ab engages the teeth of ratchet ring 236 to minimize counterclockwise movement of rotatable ring 214 that would result in loosening of sutures 222 and 224. Rotatable ratchet member 238b is secured to bearing member 234b by pivot pin 269 located between the upper surface of bearing member 234b and the bottom surface of rotatable outer ring 230 (see Figure 21). Pivot pin 269 is positioned in the rotation bore 265 of rotatable ratchet member 238b such that rotatable ratchet member 238b can pivot about pivot pin 269.

The rotatable ratchet member 238b is held in place relative to bearing member 234b by being sandwiched between bearing member 234b and rotatable outer ring 230 (see Figure 21). Thus, during rotation of rotatable outer ring 230 (see Figure 21), bearing member 234b and rotatable ratchet member 238b are rotated in a clockwise direction about ratchet ring 236. Rotatable ratchet member 238b engages the teeth of ratchet ring 236 as rotatable ratchet member is advanced 238b in the clockwise direction. When a user discontinues rotation of the rotatable outer ring, rotatable ratchet member 238b engages the teeth of ratchet ring 236 minimizing movement of rotatable outer ring 230 in a counterclockwise direction that would otherwise loosen the sutures.

The end of rotatable ratchet member 238b positioned opposite the rotation bore 265 and pivot pin includes a ratchet member engagement spring 278 and rotatable ratchet member teeth 276. Rotatable ratchet member teeth 276 engage the ratchet ring teeth 274 to minimize movement of the rotatable outer ring and bearing member 234b in counterclockwise direction. Ratchet member engagement spring 278 is provided by the cutaway portion in the head of rotatable ratchet member 238b. The nature of the material from which the head of rotatable ratchet member 238b is constructed provides sufficient resilience to undergo deformation while maintaining contact between rotatable ratchet member teeth 276 and ratchet ring teeth 274.

As the rotatable ratchet member teeth 276 slide over ratchet ring teeth 274, ratchet member engagement spring 278 flexes slightly to maintain contact between rotatable ratchet member teeth 276 and ratchet ring teeth 274. This is caused by the ramp-like configuration of rotatable ratchet member teeth 276 and ratchet ring teeth 274. When the rotatable ratchet member teeth 276 pass over the outer most ridge of ratchet ring teeth 274 such that they engage new teeth, ratchet member engagement spring 278 forces the rotatable ratchet member teeth 276 toward the center of the anchor device 210, thus maintaining engagement with ratchet ring teeth 274.

As will be appreciated by those skilled in the art, a variety of types and configurations of rotatable ratchet members can be utilized without departing from the scope of the present invention. For example, in one embodiment, the rotatable ratchet members prevent rotation of a rotatable ring in a clockwise direction. In another embodiment, the rotatable ratchet members prevent backward movement of a nonrotational actuation member. In another embodiment, a secondary spring separate from the body of the rotatable ratchet member provides the spring movement of all or part of the rotatable ratchet member.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes that come within the meaning and range of equivalency of the claims are to be embraced within their scope.

## Claims

1. A catheter anchor device, comprising:
an adhesive layer (12) attachable to the skin of a patient and defining an aperture (47) configured to be disposed about a catheter insertion site (44) on the patient;
a base (72; 90; 132; 232) coupled to the adhesive layer (12) and defining an aperture substantially aligned with the aperture of the adhesive layer; and
one or more sutures (46, 48, 50);
**characterized in that**
the base (72; 90, 132; 232) has a recess (51) formed therein, the one or more sutures (46, 48, 50) being positioned within the recess (51); and
a movable member (65; 92, 114; 214) is operably disposed with respect to the base (72; 90; 132; 232) such that movement of the movable member (65; 92; 114; 214) relative to the base (72; 90; 132; 232) results in automatic deployment of the one or more sutures (46, 48, 50) from the recess (51).

2. The catheter anchor device as recited in claim 1, further comprising at least one bearing member (134; 234) positioned between the base (132; 232) and the movable member (114; 214) and having friction reduction properties so as to facilitate rotation of the movable member (114; 214) relative to the base (132; 232).

3. The catheter anchor device as recited in claim 1, wherein one end of each of the one or more sutures is attached to the movable member (65), such that rotation of the movable member (65) with respect to the base (72) corresponds with an incremental tightening of the one or more sutures about the catheter.

4. The catheter anchor device as recited in claim 1 or 2, further comprising a ratchet mechanism (136, 138; 236, 238) utilized in connection with the movable member (114; 214), the ratchet mechanism (136, 138; 236, 238) allowing movement of the movable member (114; 214) in a first direction while securing the rotational position of the movable member (114; 214) against movement in a second direction.

5. The catheter anchor device as recited in claim 4, wherein the ratchet mechanism (236, 238) includes a ratchet ring (236) attached to the base (232), and further includes ratchet members (238) attached to the movable member (214), the ratchet ring (236) and ratchet members (238) being maintained in cooperative engagement with each other so as to allow movement of the movable member (214) in a first direction while securing the rotational position of the movable member (214) against movement in a second direction.

6. The catheter anchor device as recited in claim 5, wherein the ratchet members (238) each comprise a pivot point (265) at one end and an engagement spring (287) on the other end to maintain contact between the one or more teeth of the ratchet members (238) and stationary teeth of the ratchet ring (236).

7. The catheter anchor device as recited in claim 6, wherein the engagement spring (278) comprises a resilient component of the head of the associated ratchet member (238) which deforms as the teeth of the ratchet member (238) pass over the stationary teeth.

8. The catheter anchor device as recited in any one of claims 1-7, wherein the movable member includes a plurality of gripping members (142; 242) to facilitate gripping and rotation of the rotatable ring.

9. The catheter anchor device as recited in any one of claims 1-8, wherein the one or more sutures secure the catheter at a plurality of positions along a length of the catheter.

10. The catheter anchor device as recited in any one of claims 1-9, wherein the one or more sutures comprise first and second looped sutures (46, 48) configured to tighten about a first securement point of the catheter.

11. The catheter anchor device as recited in claim 10, wherein the one or more sutures further comprise at least a third looped suture (50) configured to slidably secure a second securement point of the catheter.

12. The catheter anchor device as recited in any one of claims 1-11, further comprising a locking mechanism (80; 136, 138; 236, 238) adapted to secure a rotational position of the movable member.

13. The catheter anchor device as recited in any one of claims 1-12, further comprising a removable lid (168) adapted to substantially cover the catheter insertion site.

14. The catheter anchor device as recited in any one of claims 1-13, further comprising an O-ring (228) located so as to maintain the position of the one or more sutures relative to the other components of the catheter anchor device prior to deployment of the one or more sutures.

15. The catheter anchor device of claim 14, wherein the recess is implemented as a suture storage channel (240), the O-ring (228) being located so as to maintain the sutures in the suture storage channel (240) prior to deployment of the sutures.

16. The catheter anchor device as recited in any one of claims 1-15, wherein the movable member comprises a rotatable ring.

17. The catheter anchor device as recited in any one of claims 1-16, further comprising a resilient flange (53) located proximate the recess (51) and arranged to facilitate retention of the sutures in the recess (51) prior to deployment of the sutures.

18. The catheter anchor device as recited in claim 1, wherein the movable member (92) and the base (90) are rectangular in shape.

19. The catheter anchor device as recited in any one of claims 1-17, further comprising an extension saddle (144) configured and arranged to support the catheter and to aid in management of the suture tag ends (120a, 120b).

20. A method of manufacturing a catheter anchor device, the method comprising:
providing an adhesive layer configured to be secured to a skin of a patient proximate a catheter insertion site;
securing a base (72; 90; 132; 232) to the adhesive layer (12);
mounting a movable member (65; 92; 114; 214) on the base (72; 90; 132; 232) such that the movable member (65; 92; 114) can be moved relative to the base (72; 90; 132; 232); and
loading one or more sutures (46, 48, 50), the one or more sutures being coupled to the movable member (65; 92; 114; 214) such that motion of the movable member (65; 92; 114; 214) in a first direction corresponds with an incremental tightening of the one or more sutures about a catheter.

21. The method of claim 20, further comprising positioning an O-ring (228) between the base and the movable member (114; 214) to facilitate securement of the one or more sutures before deployment of the sutures.

22. The method of claim 20 or 21, wherein loading of the one or more sutures is performed in connection with the use of a suture loading cylinder (272).

23. The method of claim 22, wherein the base (72; 132; 232) is positioned on a loading block (270) and the suture loading cylinder (272) is positioned inside one or more of the base (72; 132; 232) and the loading block (270).

24. The method of claim 22 or 23, wherein the loops of the sutures (222) are formed around the suture loading cylinder (272) before being loaded into the base (72; 132; 232).

25. The method of any one of claims 20-24, wherein one or more of the components of the catheter anchor device are welded during manufacture.

26. The method of any one of claims 20-25, further comprising positioning one or more bearing members (234a, 234b) relative to the base (232) to facilitate ease of movement of the movable member relative to the base, the sutures each being attached to one of the one or more bearing members (234a, 234b).

27. The method of claim 26, wherein the movable member is welded to the one or more bearing members.

28. The method of claim 26, further comprising producing one or more access bores (268) in the base so as to facilitate welding of the movable member to the one or more bearing members.

29. The method of any one of claims 26-28, further comprising securing one or more ratchet members (238) relative to the other components of the catheter anchor device by positioning the one or more ratchet members (238) between the bearing members (234a, 234b) and the movable member (232).

## Patentansprüche

1. Katheterverankerungsvorrichtung umfassend:
eine haftfähige Schicht (12), welche an die Haut eines Patienten anbringbar ist und eine Öffnung (47) definiert, welche derart ausgestaltet ist, dass sie über eine Kathetereinführungsstelle (44) auf dem Patienten anzubringen ist;
eine Basis (72; 90; 132; 232), welche an die haftfähige Schicht (12) gekoppelt ist und eine Öffnung definiert, welche im Wesentlichen mit der Öffnung der haftfähigen Schicht ausgerichtet ist; und
einen oder mehrere Fäden (46, 48, 50);
**dadurch gekennzeichnet,**
**dass** die Basis (72; 90; 132; 232) eine Vertiefung (51), welcher darin ausgebildet ist, aufweist, wobei der eine oder die mehrere Fäden (46, 48, 50) innerhalb der Vertiefung (51) angeordnet sind; und
**dass** ein bewegbares Teil (65; 92; 114; 214) bezüglich der Basis (72; 90; 132; 232) betriebsfähig angeordnet ist, so dass die Bewegung des bewegbaren Teils (65; 92; 114; 214) relativ zu der Basis (72; 90; 132; 232) zu einem automatischen Einsetzen des einen oder der mehreren Fäden (46, 48, 50) von der Vertiefung (51) führt.

2. Katheterverankerungsvorrichtung nach Anspruch 1,
**dadurch gekennzeichnet,**
**dass** die Katheterverankerungsvorrichtung darüber hinaus mindestens ein Lagerungsteil (134; 234) umfasst, welches zwischen der Basis (132; 232) und dem bewegbaren Teil (114; 214) angeordnet ist und eine Reibung verringernde Eigenschaften aufweist, um so eine Drehung des bewegbaren Teils (114; 234) relativ zu der Basis (132; 232) zu ermöglichen.

3. Katheterverankerungsvorrichtung nach Anspruch 1,
**dadurch gekennzeichnet,**
**dass** ein Ende von jedem des einen oder der mehreren Fäden an dem bewegbaren Teil (65) angebracht ist, so dass eine Drehung des bewegbaren Teils (65) bezüglich der Basis (72) einem inkrementellen Straffen des einen oder der mehreren Fäden bezüglich des Katheters entspricht.

4. Katheterverankerungsvorrichtung nach Anspruch 1 oder 2,
**dadurch gekennzeichnet,**
**dass** die Katheterverankerungsvorrichtung darüber hinaus einen Sperrmechanismus (136, 138; 236, 238) umfasst, welcher in Verbindung mit dem bewegbaren Teil (114; 214) eingesetzt wird, wobei der Sperrmechanismus (136, 138; 236, 238) eine Bewegung des bewegbaren Teils (114; 214) in eine erste Richtung ermöglicht während die Rotationsposition des bewegbaren Teils (114; 214) gegen eine Bewegung in eine zweite Richtung gesichert ist.

5. Katheterverankerungsvorrichtung nach Anspruch 4,
**dadurch gekennzeichnet,**
**dass** der Sperrmechanismus (236, 238) einen Sperrring (236) aufweist, welcher an der Basis (232) befestigt ist, und darüber hinaus Sperrteile (238) umfasst, welche an dem bewegbaren Teil (214) angebracht sind, wobei der Sperrring (236) und die Sperrteile (238) in einem zusammenwirkenden Eingriff miteinander gehalten werden, um so eine Bewegung des bewegbaren Teils (214) in eine erste Richtung zu ermöglichen, während die Rotationsposition des bewegbaren Teils (214) gegen eine Bewegung in eine zweite Richtung gesichert ist.

6. Katheterverankerungsvorrichtung nach Anspruch 5,
**dadurch gekennzeichnet,**
**dass** die Sperrteile (238) jeweils einen Drehpunkt (265) an einem Ende und eine Eingriffsfeder (287) an dem anderen Ende umfassen, um einen Kontakt zwischen dem einen oder den mehreren Zähnen der Sperrteile (238) und einem ortsfesten Zahn des Sperrrings (236) zu halten.

7. Katheterverankerungsvorrichtung nach Anspruch 6,
**dadurch gekennzeichnet,**
**dass** die Eingriffsfeder (278) eine nachgiebige Komponente des Kopfes des zugehörigen Sperrteils (238) umfasst, welche sich verformt, wenn die Zähne des Sperrteils (238) über die ortsfesten Zähne verlaufen.

8. Katheterverankerungsvorrichtung nach einem der Ansprüche 1 bis 7,
**dadurch gekennzeichnet,**
**dass** das bewegbare Teil mehrere Griffteile (142; 242) umfasst, um ein Greifen und ein Drehen des drehbaren Rings zu ermöglichen.

9. Katheterverankerungsvorrichtung nach einem der Ansprüche 1 bis 8,
**dadurch gekennzeichnet,**
**dass** der eine oder die mehreren Fäden den Katheter an mehreren Stellen entlang einer Länge des Katheters befestigen.

10. Katheterverankerungsvorrichtung nach einem der Ansprüche 1 bis 9,
**dadurch gekennzeichnet,**
**dass** der eine oder die mehreren Fäden einen ersten und einen zweiten schleifenförmigen Faden (46, 48) umfassen, welche derart ausgestaltet sind, dass sie gegenüber einem ersten Befestigungspunkt des Katheters spannen.

11. Katheterverankerungsvorrichtung nach Anspruch 10,
**dadurch gekennzeichnet,**
**dass** der eine oder die mehreren Fäden darüber hinaus mindestens einen dritten schleifenförmigen Faden (50) umfassen, welcher derart ausgestaltet ist, dass er gleitend einen zweiten Befestigungspunkt des Katheters befestigt.

12. Katheterverankerungsvorrichtung nach einem der Ansprüche 1 bis 11,
**dadurch gekennzeichnet,**
**dass** die Katheterverankerungsvorrichtung darüber hinaus einen Verriegelungsmechanismus (80; 136, 138; 236, 238) umfasst, welcher derart ausgestaltet ist, dass er eine Rotationsposition des bewegbaren Teils sichert.

13. Katheterverankerungsvorrichtung nach einem der Ansprüche 1 bis 12,
**dadurch gekennzeichnet,**
**dass** die Katheterverankerungsvorrichtung darüber hinaus einen entfernbaren Deckel (168) umfasst, welcher derart ausgestaltet ist, dass er die Einführungsstelle des Katheters im Wesentlichen abdeckt.

14. Katheterverankerungsvorrichtung nach einem der Ansprüche 1 bis 13,
**dadurch gekennzeichnet,**
**dass** die Katheterverankerungsvorrichtung darüber hinaus einen O-Ring (228) umfasst, welcher derart angeordnet ist, dass er die Position des einen oder der mehreren Fäden relativ zu den anderen Komponenten der Katheterverankerungsvorrichtung vor dem Einsatz des einen oder der mehreren Fäden hält.

15. Katheterverankerungsvorrichtung nach Anspruch 14,
**dadurch gekennzeichnet,**
**dass** die Vertiefung als ein Fadenaufbewahrungskanal (240) ausgebildet ist, wobei der O-Ring (228) derart angeordnet ist, dass er die Fäden in dem Fadenaufbewahrungskanal (240) vor dem Einsatz der Fäden hält.

16. Katheterverankerungsvorrichtung nach einem der Ansprüche 1 bis 15,
**dadurch gekennzeichnet,**
**dass** das bewegbare Teil einen drehbaren Ring umfasst.

17. Katheterverankerungsvorrichtung nach einem der Ansprüche 1 bis 16,
**dadurch gekennzeichnet,**
**dass** die Katheterverankerungsvorrichtung darüber hinaus einen nachgiebigen Flansch (53) umfasst, welcher in der Nähe der Vertiefung (51) angeordnet ist und derart angeordnet ist, dass er ein Zurückhalten der Fäden in der Vertiefung (51) vor dem Einsatz der Fäden ermöglicht.

18. Katheterverankerungsvorrichtung nach Anspruch 1,
**dadurch gekennzeichnet,**
**dass** das bewegbare Teil (92) und die Basis (90) eine rechteckige Form aufweisen.

19. Katheterverankerungsvorrichtung nach einem der Ansprüche 1 bis 17,
**dadurch gekennzeichnet,**
**dass** die Katheterverankerungsvorrichtung darüber hinaus eine Verlängerungsschelle (144) umfasst, welche derart ausgestaltet und angeordnet ist, dass sie den Katheter hält und eine Handhabung der Fadenenden (120a, 120b) unterstützt.

20. Verfahren zur Herstellung einer Katheterverankerungsvorrichtung, wobei das Verfahren umfasst:
Bereitstellen einer haftfähigen Schicht, welche derart ausgestaltet ist, dass sie an einer Haut eines Patienten in der Nähe einer Kathetereinführungsstelle zu befestigen ist;
Befestigen einer Basis (72; 90; 132; 232) an der haftfähigen Schicht (12);
Anbringen eines bewegbaren Teils (65; 92; 114; 214) an der Basis (72; 90; 132; 232), so dass das bewegbare Teil (65; 92; 114; 214) relativ zu der Basis (72; 90; 132; 232) bewegbar ist; und
Zuführen von einem oder von mehreren Fäden (46, 48, 50), wobei der eine oder die mehreren Fäden mit dem bewegbaren Teil (65; 92; 114; 214) gekoppelt sind, so dass eine Bewegung des bewegbaren Teils (65; 92; 114; 214) in eine erste Richtung einem inkrementellen Straffen des einen oder der mehreren Fäden bezüglich eines Katheters entspricht.

21. Verfahren nach Anspruch 20,
**dadurch gekennzeichnet,**
**dass** das Verfahren darüber hinaus ein Anordnen eines O-Rings (228) zwischen der Basis und dem bewegbaren Teil (114; 214) umfasst, um eine Befestigung des einen oder der mehreren Fäden vor einem Einsatz der Fäden zu ermöglichen.

22. Verfahren nach Anspruch 20 oder 21,
**dadurch gekennzeichnet,**
**dass** das Zuführen des einen oder der mehreren Fäden in Verbindung mit der Verwendung eines Fadenzufuhrzylinders (272) durchgeführt wird.

23. Verfahren nach Anspruch 22,
**dadurch gekennzeichnet,**
**dass** die Basis (72; 132; 232) auf einem Zufuhrblock (270) angeordnet wird, und dass der Fadenzufuhrzylinder (272) innerhalb der Basis (72; 132; 232) und/oder des Zufuhrblocks (270) angeordnet wird.

24. Verfahren nach Anspruch 22 oder 23,
**dadurch gekennzeichnet,**
**dass** die Schleifen der Fäden (222) um den Fadenzufuhrzylinder (272) herum ausgebildet werden, bevor sie in die Basis (72; 132; 232) zugeführt werden.

25. Verfahren nach einem der Ansprüche 20 bis 24,
**dadurch gekennzeichnet,**
**dass** eine oder mehrere der Komponenten der Katheterverankerungsvorrichtung während der Herstellung geschweißt werden.

26. Verfahren nach einem der Ansprüche 20 bis 25,
**dadurch gekennzeichnet,**
**dass** das Verfahren darüber hinaus ein Anordnen von einem oder von mehreren Lagerteilen (234a, 234b) relativ zu der Basis (232) umfasst, um eine einfache Bewegung des bewegbaren Teils relativ zu der Basis zu ermöglichen, wobei die Fäden jeweils an einem der einen oder mehreren Lagerteile (234a, 234b) angebracht werden.

27. Verfahren nach Anspruch 26,
**dadurch gekennzeichnet,**
**dass** das bewegbare Teil an das eine oder die mehreren Lagerteile geschweißt wird.

28. Verfahren nach Anspruch 26,
**dadurch gekennzeichnet,**
**dass** das Verfahren darüber hinaus ein Ausbilden von einem oder von mehreren Zugangslöchern (268) in der Basis umfasst, um so ein Schweißen des beweglichen Teils an das eine oder die mehreren Lagerteile zu ermöglichen.

29. Verfahren nach einem der Ansprüche 26 bis 28,
**dadurch gekennzeichnet,**
**dass** das Verfahren darüber hinaus ein Befestigen von einem oder von mehreren Sperrteilen (238) relativ zu den anderen Komponenten der Katheterverankerungsvorrichtung umfasst, indem das eine oder die mehreren Sperrteile (238) zwischen den Lagerteilen (234a, 234b) und dem bewegbaren Teil (232) angeordnet werden.

## Revendications

1. Dispositif d'ancrage pour cathéter, qui comprend :
une couche adhésive (12) pouvant être attachée à la peau d'un patient et définissant une ouverture (47) configurée pour être disposée autour d'un site d'insertion de cathéter (44) sur le patient ;
une base (72 ; 90 ; 132 ; 232) couplée à la couche adhésive (12) et définissant une ouverture sensiblement alignée sur l'ouverture de la couche adhésive ; et
une ou plusieurs sutures (46, 48, 50) ;
**caractérisé en ce que** :
une cavité (51) est formée à l'intérieur de la base (72 ; 90 ; 132 ; 232), la ou les sutures (46, 48, 50) étant positionnées à l'intérieur de la cavité (51) ; et
un élément amovible (65 ; 92 ; 114 ; 214) est disposé de manière opérationnelle relativement à la base (72 ; 90 ; 132 ; 232) de telle manière que le mouvement de l'élément amovible (65 ; 92 ; 114 ; 214) par rapport à la base (72 ; 90 ; 132 ; 232) résulte en le déploiement automatique de la ou des sutures (46, 48, 50) de la cavité (51).

2. Dispositif d'ancrage pour cathéter selon la revendication 1, qui comprend en outre au moins un élément de roulement (134 ; 234) positionné entre la base (132 ; 232) et l'élément amovible (114 ; 214) et ayant des propriétés de réduction de la friction de manière à faciliter la rotation de l'élément amovible (114 ; 214) par rapport à la base (132 ; 232).

3. Dispositif d'ancrage pour cathéter selon la revendication 1, dans lequel une extrémité de chacune de la ou des sutures est attachée à l'élément amovible (65), de telle sorte que la rotation de l'élément amovible (65) relativement à la base (72) correspond à un serrage incrémentiel de la ou des sutures autour du cathéter.

4. Dispositif d'ancrage pour cathéter selon la revendication 1 ou 2, qui comprend en outre un mécanisme d'encliquetage (136, 138 ; 236, 238) utilisé relativement à l'élément amovible (114 ; 214), le mécanisme d'encliquetage (136, 138 ; 236, 238) permettant le mouvement de l'élément amovible (114 ; 214) dans une première direction tout en préservant la position rotative de l'élément amovible (114 ; 214) d'un mouvement dans une deuxième direction.

5. Dispositif d'ancrage pour cathéter selon la revendication 4, dans lequel le mécanisme d'encliquetage (236, 238) comprend une bague d'encliquetage (236) fixée à la base (232), et comprend en outre des éléments d'encliquetage (238) fixés à l'élément amovible (214), la bague d'encliquetage (236) et les éléments d'encliquetage (238) étant maintenus dans un accouplement coopératif les uns avec les autres de manière à permettre le mouvement de l'élément amovible (214) dans une première direction tout en préservant la position rotationnelle de l'élément amovible (214) d'un mouvement dans une deuxième direction.

6. Dispositif d'ancrage pour cathéter selon la revendication 5, dans lequel les éléments d'encliquetage (238) comprennent chacun un point de pivot (265) au niveau d'une extrémité et un ressort d'accouplement (287) sur l'autre extrémité pour maintenir le contact entre la ou les dents des éléments d'encliquetage (238) et les dents stationnaires de la bague d'encliquetage (236).

7. Dispositif d'ancrage pour cathéter selon la revendication 6, dans lequel le ressort d'accouplement (278) comprend un composant résilient de la tête de l'élément d'encliquetage associé (238) qui se déforme lorsque les dents de l'élément d'encliquetage (238) passent sur les dents stationnaires.

8. Dispositif d'ancrage pour cathéter selon l'une quelconque des revendications 1 à 7, dans lequel l'élément amovible comprend une pluralité d'éléments de préhension (142 ; 242) destinés à faciliter la préhension et la rotation de la bague rotative.

9. Dispositif d'ancrage pour cathéter selon l'une quelconque des revendications 1 à 8, dans lequel la ou les sutures fixent le cathéter à une pluralité de positions le long d'une longueur du cathéter.

10. Dispositif d'ancrage pour cathéter selon l'une quelconque des revendications 1 à 9, dans lequel la ou les sutures comprennent des première et deuxième sutures en boucle (46, 48) configurées pour se serrer autour d'un premier point de fixation du cathéter.

11. Dispositif d'ancrage pour cathéter selon la revendication 10, dans lequel la ou les sutures comprennent en outre au moins une troisième suture en boucle (50) configurée pour fixer par coulissement un deuxième point de fixation du cathéter.

12. Dispositif d'ancrage pour cathéter selon l'une quelconque des revendications 1 à 11, qui comprend en outre un mécanisme de verrouillage (80 ; 136 ; 138 ; 236 ; 238) adapté pour fixer une position rotative de l'élément amovible.

13. Dispositif d'ancrage pour cathéter selon l'une quelconque des revendications 1 à 12, qui comprend en outre un couvercle amovible (168) adapté pour sensiblement couvrir le site d'insertion du cathéter.

14. Dispositif d'ancrage pour cathéter selon l'une quelconque des revendications 1 à 13, qui comprend en outre un joint torique (228) situé de manière à maintenir la position de la ou des sutures par rapport aux autres composants du dispositif d'ancrage pour cathéter avant le déploiement de la ou des sutures.

15. Dispositif d'ancrage pour cathéter selon la revendication 14, dans lequel la cavité est réalisée sous la forme d'un canal de stockage de sutures (240), le joint torique (228) étant situé de manière à maintenir les sutures dans le canal de stockage de sutures (240) avant le déploiement des sutures.

16. Dispositif d'ancrage pour cathéter selon l'une quelconque des revendications 1 à 15, dans lequel l'élément amovible comprend une bague rotative.

17. Dispositif d'ancrage pour cathéter selon l'une quelconque des revendications 1 à 16, qui comprend en outre une bride élastique (53) située à proximité de la cavité (51) et configurée pour faciliter la rétention des sutures dans la cavité (51) avant le déploiement des sutures.

18. Dispositif d'ancrage pour cathéter selon la revendication 1, dans lequel l'élément amovible (92) et la base (90) sont de forme rectangulaire.

19. Dispositif d'ancrage pour cathéter selon l'une quelconque des revendications 1 à 17, comprenant en outre une selle à extension (144) configurée et disposée pour supporter le cathéter et pour aider à prendre en charge les extrémités des sutures (120a, 120b).

20. Procédé de fabrication d'un dispositif d'ancrage pour cathéter, le procédé comprenant :
la fourniture d'une couche adhésive configurée pour être fixée à la peau d'un patient à proximité d'un site d'insertion de cathéter ;
la fixation d'une base (72 ; 90 ; 132 ; 232) à la couche adhésive (12) ;
le montage d'un élément amovible (65 ; 92 ; 114 ; 214) sur la base (72 ; 90 ; 132 ; 232) de manière à ce que l'élément amovible (65 ; 92 ; 114) puisse être déplacé relativement à la base (72 ; 90 ; 132 ; 232) ; et
le chargement d'une ou plusieurs sutures (46, 48, 50), la ou les sutures étant couplées à l'élément amovible (65 ; 92 ; 114 ; 214) de telle manière que le mouvement de l'élément amovible (65 ; 92 ; 114 ; 214) dans une première direction correspond à un serrage incrémentiel de la ou des sutures autour d'un cathéter.

21. Procédé selon la revendication 20, comprenant en outre le positionnement d'un joint torique (228) entre la base et l'élément amovible (114 ; 214) pour faciliter la fixation de la ou des sutures avant le déploiement des sutures.

22. Procédé selon la revendication 20 ou 21, dans lequel le chargement de la ou des sutures est réalisé relativement à l'utilisation d'un cylindre de chargement de sutures (272).

23. Procédé selon la revendication 22, dans lequel la base (72 ; 132 ; 232) est positionnée sur un bloc de chargement (270) et le cylindre de chargement de suture (272) est positionné à l'intérieur de la base (72 ; 132 ; 232) et/ou du bloc de chargement (270).

24. Procédé selon la revendication 22 ou 23, dans lequel les boucles des sutures (222) sont formées autour du cylindre de chargement de sutures (272) avant d'être chargées dans la base (72 ; 132 ; 232).

25. Procédé selon l'une quelconque des revendications 20 à 24, dans lequel un ou plusieurs des composants du dispositif d'ancrage pour cathéter sont soudés durant la fabrication.

26. Procédé selon l'une quelconque des revendications 20 à 25, comprenant en outre le positionnement du ou des éléments de roulement (234a, 234b) relativement à la base (232) pour faciliter le mouvement de l'élément amovible par rapport à la base, les sutures étant chacune attachées à l'un du ou des éléments de roulement (234a, 234b).

27. Procédé selon la revendication 26, dans lequel l'élément amovible est soudé au ou aux éléments de roulement.

28. Procédé selon la revendication 26, comprenant en outre la production d'un ou de plusieurs canaux d'accès (268) dans la base pour faciliter la soudure de l'élément amovible au ou aux éléments de roulement.

29. Procédé selon l'une quelconque des revendications 26 à 28, comprenant en outre la fixation d'un élément d'encliquetage (238) ou plus relativement aux autres composants du dispositif d'ancrage pour cathéter par positionnement du ou des éléments d'encliquetage (238) entre les éléments de roulement (234a, 234b) et l'élément amovible (232).
